(19) Europäisches Patentamt
European Patent Office
Office européen des brevets

(11) **EP 4 065 191 B1**

(12) **EUROPÄISCHE PATENTSCHRIFT**

(45) Veröffentlichungstag und Bekanntmachung des Hinweises auf die Patenterteilung:
**26.04.2023   Patentblatt 2023/17**

(21) Anmeldenummer: **21766623.9**

(22) Anmeldetag: **24.08.2021**

(51) Internationale Patentklassifikation (IPC):
***A61M 1/36*** (2006.01)

(52) Gemeinsame Patentklassifikation (CPC):
**A61M 1/3609; A61M 1/3646;** A61M 1/3649;
A61M 1/365; A61M 2205/3306; A61M 2230/207

(86) Internationale Anmeldenummer:
**PCT/EP2021/073338**

(87) Internationale Veröffentlichungsnummer:
**WO 2022/048943 (10.03.2022 Gazette 2022/10)**

(54) **AUTOMATISCHE REINFUSION VON BLUT NACH EINER BLUTBEHANDLUNGSTHERAPIE**

AUTOMATIC REINFUSION OF BLOOD FOLLOWING A BLOOD TREATMENT THERAPY

REPERFUSION AUTOMATIQUE DE SANG À LA SUITE D'UNE THÉRAPIE DE TRAITEMENT SANGUIN

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.09.2020   DE 102020122936**

(43) Veröffentlichungstag der Anmeldung:
**05.10.2022   Patentblatt 2022/40**

(73) Patentinhaber: **B. Braun Avitum AG**
**34212 Melsungen (DE)**

(72) Erfinder:
• **SCHILLER, Joana Sophie**
  **48429 Rheine (DE)**
• **WÜRSCHMIDT, Tobias**
  **34346 Hann. Münden (DE)**

(74) Vertreter: **Winter, Brandl - Partnerschaft mbB**
**Alois-Steinecker-Straße 22**
**85354 Freising (DE)**

(56) Entgegenhaltungen:
EP-A1- 2 583 701          DE-A1- 3 535 036
DE-A1-102010 025 516    US-A1- 2017 361 004

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Offenbarung betrifft eine extrakorporale Blutbehandlungsvorrichtung, insbesondere Dialysemaschine, welche für eine automatische Reinfusion/ Wiederzuführung von Blut zu einem Patienten nach einer Blutbehandlungstherapie vorbereitet bzw. eingerichtet ist. Weiterhin betrifft die vorliegende Offenbarung ein Verfahren zum automatischen Durchführen einer Reinfusion von Blut nach einer Blutbehandlungstherapie.

Technischer Hintergrund

Nach einer erfolgten Blutbehandlungstherapie, in welcher Blut eines Patienten unter Verwendung eines Dialysators und einer den Dialysator durchströmenden Dialysierflüssigkeit extrakorporal gereinigt wurde, wird grundsätzlich das Blut des Patienten, welches sich noch in einem extrakorporalen Kreislauf, beispielsweise in einem arteriellen und venösen Schlauchsystem (A/V-Schlauchsystem), befindet, dem Patienten wiederzugeführt. Dieser Vorgang wird als Reinfusion bezeichnet. Erst wenn das in dem extrakorporalen Kreislauf noch vorhandene Blut dem Patienten in geeigneter Weise wiederzugeführt/ reinfundiert wurde, wird der Patient von dem extrakorporalen Schlauchsystem vollständig abgekoppelt.

Stand der Technik

Aus dem Stand der Technik ist beispielsweise ein Reinfusionsverfahren bekannt, das eine isotonische Kochsalzlösung verwendet, die aus Beuteln zugeführt wird (in der Folge kurz als Kochsalzbeutel bezeichnet). Dabei wird nach einer erfolgten Blutbehandlungstherapie von einem Anwender/ Pflegepersonal zunächst lediglich das arterielle Ende/ der arterielle Abschnitt des extrakorporalen Kreislaufs/ Schlauchsystems von dem Patienten abgekoppelt und mit einem Kochsalzbeutel verbunden. Wenn sich nun eine in dem extrakorporalen Kreislauf vorhandene Blutpumpe in Therapierichtung dreht, wird das Blut durch die Kochsalzlösung verdrängt und durchströmt den arteriellen Abschnitt, einen Dialysator und einen venösen Abschnitt des extrakorporalen Kreislaufs, und wird über das venöse Ende des extrakorporalen Kreislaufs dem Patienten zurückgegeben.

Weiterhin ist aus dem Stand der Technik ein Reinfusionsverfahren über einen Substituatport bzw. -anschluss bekannt. Dabei wir nach einer erfolgten Blutbehandlungstherapie von einem Anwender auch zunächst lediglich das arterielle Ende/ der arterielle Abschnitt des extrakorporalen Kreislaufs/ Schlauchsystems von dem Patienten abgekoppelt und mit einem Substituatport bzw. -anschluss der extrakorporalen Blutbehandlungsvorrichtung verbunden. Wenn sich in diesem Fall die Blutpumpe in Therapierichtung dreht, wird das Blut durch die Substitutionsflüssigkeit verdrängt und durchströmt den arteriellen Abschnitt, einen Dialysator und einen venösen Abschnitt des extrakorporalen Kreislaufs, und wird über das venöse Ende des extrakorporalen Kreislaufs dem Patienten zurückgegeben.

Bei beiden genannten Reinfusionsverfahren kann die Reinfusion durch ein Stoppen der Blutpumpe abgebrochen werden. Ein Abbruch der Reinfusion kann beispielsweise volumengesteuert vorgenommen werden, beispielweise wenn ein vorbestimmtes Volumen reinfundiert wurde. Weiterhin kann ein Abbruch der Reinfusion zeitgesteuert vorgenommen werden, beispielsweise wenn eine vorbestimmte Reinfusionszeit verstrichen ist. Darüber hinaus kann grundsätzlich auch an dem venösen Ende/ in dem venösen Abschnitt ein Sensor vorgesehen sein, welcher eingerichtet ist, eine Kochsalzlösung oder eine Substitutionsflüssigkeit zu erkennen. Beispielsweise ist aus der EP 1 996 253 B1 bekannt, die Reinfusion durch ein Anhalten der Blutpumpe abzubrechen, wenn eine Phasengrenze zwischen Blut und Kochsalzlösung das venöse Ende des extrakorporalen Kreislaufs erreicht hat.

In nachteilhafter Weise muss bei den beiden genannten Reinfusionsverfahren entweder ein Substituatport an der Dialysemaschine vorhanden sein oder ein externer Kochsalzbeutel verwendet werden. Maschinen ohne integrierten Substituatanschluss müssen in anderen Worten zwangsläufig einen Kochsalzbeutel für die Reinfusion verwenden, was mit hohen Kosten (für den Kochsalzbeutel) verbunden ist. Außerdem muss ein Anwender bei den genannten Reinfusionsverfahren zumindest zweimal aktiv eingreifen. Insbesondere wird der Patient bei diesen Verfahren zunächst arteriell abgekoppelt (Aktion des Anwenders), dann erfolgt die Reinfusion, und abschließend wird der Patient venös abgekoppelt (Aktion des Anwenders).

Es ist grundsätzlich wünschenswert, Interaktionen bzw. Eingriffe des Anwenders auf ein Minimum zu beschränken. Ferner wäre es von Vorteil, wenn die Reinfusion ohne externen Kochsalzbeutel oder ohne Substituatport durchgeführt werden kann. Insbesondere wäre es wünschenswert, eine automatische Reinfusion von Blut nach einer Blutbehandlungstherapie durchzuführen, welche keinen manuellen Eingriff des Anwenders zwischen dem Ende der Blutbehandlungstherapie und dem Beginn der Reinfusion erfordert.

Dabei ist es aus dem Stand der Technik grundsätzlich schon bekannt, eine Reinfusion von Blut automatisch und unmittelbar nach der Blutbehandlungstherapie derart zu steuern, dass eine Dialysierflüssigkeit von einem Dialysierflüssigkeitskreislauf über eine Membran eines Dialysators an den extrakorporalen Kreislauf geliefert wird, welche bei der Reinfusion das in dem extrakorporalen Kreislauf vorhandene Blut zu einem Patienten hin verdrängt, um das Blut dem Patienten zurückzugeben. In diesem Zusammenhang wird lediglich beispielhaft auf die EP 2 950 841 B1, die EP 2 583 702 B1, die EP 3 476 414 A1 oder die EP 1 684 825 B1 verwiesen. Aus der EP 2 950 841 B1 und der EP 2 583 702 B1 ist dabei ein volumengesteuerter Abbruch der Reinfusion bekannt. Dabei wird in der EP 2 583 702 B1 das volumengesteuerte Beenden/ Abbrechen der Reinfusion gestartet, wenn ein bereitgestellter Farbsensor Dialysierflüssigkeit erkennt. Weiterhin ist aus der EP 3 476 414 A1 bekannt, eine Reinfusion automatisch abzubrechen, wenn ein vorgesehener Blutsensor eine Blutkonzentration unter einem maximalen erlaubten Schwellenwert in einem arteriellen Abschnitt erfasst.

Die DE 35 35 036 A1 offenbart ferner eine extrakorporale Blutbehandlungsvorrichtung, welche Sensoren zur Strömungsüberwachung in einem extrakorporalen Kreislauf aufweist. Bei einer Reinfusion von Blut wird eine Spülflüssigkeit aus einem Behälter geliefert, welche das in dem extrakorporalen Kreislauf vorhandene Blut zu einem Patienten hin verdrängt.

Die DE 10 2010 025516 A1 offenbart eine extrakorporale Blutbehandlungsvorrichtung, welche optische Detektoren zur Erfassung einer Phasengrenze in einer einen extrakorporalen Blutkreislauf durchströmenden Flüssigkeit aufweist. Eine Reinfusion von Blut wird derart gesteuert, dass eine Dialysierflüssigkeit von einem Dialysierflüssigkeitskreislauf über eine Membran eines Dialysators an den extrakorporalen Kreislauf geliefert wird, welche das in dem extrakorporalen Kreislauf vorhandene Blut zu einem Patienten hin verdrängt. Die optischen Detektoren erkennen die Phasengrenze zwischen Blut und Prozessfluid und veranlassen ein Stoppen des Rückgabeprozesses.

Die EP 2 583 701 A1 offenbart eine extrakorporale Blutbehandlungsvorrichtung, welche Sensoren in einem extrakorporalen Kreislauf aufweist. Bei der Reinfusion von Blut wird Dialysierflüssigkeit über den Dialysator an den extrakorporalen Kreislauf geliefert, um das in dem extrakorporalen Kreislauf vorhandene Blut zum Patienten hin zu verdrängen.

Die US 2017/361004 A1 offenbart eine extrakorporale Blutbehandlungsvorrichtung mit Hämatokritsensoren in dem extrakorporalen Kreislauf und sieht eine Reinfusion von Blut vor.

Im Stand der Technik gibt es grundsätzlich Verbesserungspotential im Hinblick auf geeignete Abbruchkriterien zum Abbrechen der Reinfusion. Insbesondere stellt der Stand der Technik kein geeignetes Abbrechen der Reinfusion bereit, wenn Blut dem Patienten sowohl über den venösen Abschnitt als auch über den arteriellen Abschnitt zurückgegeben wird. Dabei berücksichtigt der Stand der Technik insbesondere nicht, dass bei der Reinfusion keine Phasengrenze zwischen Blut und Reinfusionsflüssigkeit (Dialysierflüssigkeit) vorliegt, sondern sich Blut und Reinfusionsflüssigkeit/ Dialysierflüssigkeit vermischen. Weiterhin wird im Stand der Technik nicht in zufriedenstellender Weise ein Lufteintritt in einen Patienten bei der Reinfusion verhindert.

Vor diesem Hintergrund ist es Aufgabe der vorliegenden Offenbarung, die genannten Nachteile aus dem Stand der Technik zu vermeiden oder wenigstens zu mildern. Insbesondere soll eine automatische Einleitung und Durchführung einer Reinfusion nach Abschluss einer Dialysebehandlung bereitgestellt werden, welche es ermöglicht, dass ein Anwender erst nach der Reinfusion für ein Abkoppeln des Patienten eingreifen muss. In anderen Worten soll eine Interaktion/ ein Eingreifen des Pflegepersonals auf ein Minimum beschränkt werden. Die Reinfusion soll daher insbesondere ohne externen Kochsalzbeutel bzw. ohne Substituatport durchgeführt werden. Insbesondere soll als Reinfusionsflüssigkeit Dialysierflüssigkeit verwendet werden, welche von dem Dialysierflüssigkeitskreislauf über eine Membran eines Dialysators an den extrakorporalen Kreislauf geliefert wird. Aufgabe der vorliegenden Offenbarung ist es auch, eine verbesserte Steuerung eines Abbrechens der Reinfusion bereitzustellen, wenn Blut dem Patienten sowohl über den arteriellen Abschnitt als auch über den venösen Abschnitt des extrakorporalen Kreislaufs zurückgegeben wird, und ein Eintreten von Luft in den Patienten bei der Reinfusion zu vermeiden.

## Kurzbeschreibung der Offenbarung

Die vorliegende Offenbarung stellt eine extrakorporale Blutbehandlungsvorrichtung nach Anspruch 1 bereit.

Die Offenbarung betrifft zunächst eine extrakorporale Blutbehandlungsvorrichtung mit: einem extrakorporalen Kreislauf umfassend einen arteriellen Abschnitt und einen venösen Abschnitt, einem Dialysator und einem Dialysierflüssigkeitskreislauf, wobei der extrakorporale Kreislauf und der Dialysierflüssigkeitskreislauf über eine in dem Dialysator vorgesehene Membran voneinander getrennt sind; in dem venösen Abschnitt ein erster Sensor vorgesehen ist, welcher eingerichtet ist, einen Hämatokrit-Anteil in einer den venösen Abschnitt durchströmenden Flüssigkeit zu erfassen; in dem arteriellen Abschnitt ein zweiter Sensor vorgesehen ist, welcher eingerichtet ist, einen Hämatokrit-Anteil in einer den arteriellen Abschnitt durchströmenden Flüssigkeit zu erfassen; und die extrakorporale Blutbehandlungsvorrichtung ferner eine Steuereinheit enthält, welche eingerichtet ist, eine Reinfusion von Blut (automatisch/ (unmittelbar) nach der Blutbehandlungstherapie) derart zu steuern, dass eine Dialysierflüssigkeit von dem Dialysierflüssigkeitskreislauf über die Membran des Dialysators an den extrakorporalen Kreislauf geliefert wird, welche bei der Reinfusion das in dem extrakorporalen Kreislauf vorhandene Blut zu einem Patienten hin verdrängt, um das Blut dem Patienten sowohl über den venösen Abschnitt als auch über den arteriellen Abschnitt zurückzugeben. Die Steuereinheit ist eingerichtet, die Reinfusion über den venösen Abschnitt abzubrechen, wenn von dem ersten Sensor erfasst wird oder von der Steuereinheit auf der Grundlage von von dem ersten Sensor erfassten Informationen berechnet oder vorhergesagt wird, dass der Hämatokrit-Anteil einen vorbestimmten Grenzwert unterschreitet, und die Reinfusion über den arteriellen Abschnitt abzubrechen, wenn von dem zweiten Sensor erfasst wird oder von der Steuereinheit auf der Grundlage von von dem zweiten Sensor erfassten Informationen berechnet oder vorhergesagt wird, dass der Hämatokrit-Anteil den vorbestimmten Grenzwert unterschreitet.

In dem Dialysierflüssigkeitskreislauf der extrakorporalen Blutbehandlungsvorrichtung sind bevorzugt sowohl ein Dialysatoreinlassventil als auch ein Dialysatorauslassventil vorgesehen. Das Dialysatoreinlassventil ist bevorzugt an einem Dialysierflüssigkeitszufluss stromaufwärts des Dialysators vorgesehen. Das Dialysatorauslassventil ist bevorzugt an einem Dialysierflüssigkeitsabfluss stromabwärts des Dialysators vorgesehen. In vorteilhafter Weise ist in dem Dialysierflüssigkeitszufluss (stromaufwärts des Dialysatoreinlassventils) eine Flusspumpe-Eingang angeordnet. Weiter bevorzugt ist in dem Dialysierflüssigkeitsabfluss (stromabwärts des Dialysatorauslassventils) eine Flusspumpe-Ausgang angeordnet. Die Flusspumpe-Eingang und/oder die Flusspumpe-Ausgang ist/ sind bevorzugt als Zahnradpumpe/n ausgebildet.

Gemäß der vorliegenden Offenbarung wird bevorzugt nach einem Ende der Blutbehandlungstherapie die Reinfusion über einen Überdruck in dem Dialysierflüssigkeitskreislauf bzw. auf der Dialysierflüssigkeitsseite des Dialysators eingeleitet. Durch den Überdruck tritt Dialysierflüssigkeit durch die Membran des Dialysators. Diese Dialysierflüssigkeit dient offenbarungsgemäß als Reinfusionsflüssigkeit. Der Überdruck auf der Dialysierflüssigkeitsseite wird bevorzugt durch eine geeignete Steuerung/ Regelung durch die Steuereinheit aufgebaut. Insbesondere kann die Flusspumpe-Eingang angesteuert werden, damit diese Dialysierflüssigkeit in den Dialysator pumpt. Dabei ist bevorzugt die Flusspumpe-Ausgang gestoppt. Wenn das Dialysatorauslassventil geschlossen ist, wird durch die Dialysierflüssigkeit ein geeigneter Druck (Transmembrandruck) auf die Membran des Dialysators ausgeübt. Dieser Druck sollte einen maximal zulässigen Transmembrandruck des verwendeten Dialysators nicht überschreiten. Weiterhin wird der Druck (Transmembrandruck) bevorzugt derart gewählt, dass ein Übertritt der Dialysierflüssigkeit mit einem definierten Fluss (Flussrate/ Volumenstrom) erzielt wird. Insbesondere kann der definierte Fluss in Abhängigkeit von dem verwendeten Dialysator auf einen Fluss zwischen 50 ml/min bis 200 ml/min, beispielsweise 100 ml/min eingestellt werden.

Bevorzugt ist in dem arteriellen Abschnitt des extrakorporalen Kreislaufs eine (arterielle) Blutpumpe (bevorzugt als Rollenpumpe ausgebildet, um durch Quetschen eines Schlauchs ein Fluid/ eine Flüssigkeit zu fördern) vorgesehen, welche für einen Drehrichtungswechsel eingerichtet ist, und die Steuereinheit steuert bei der Reinfusion über den arteriellen Abschnitt die arterielle Blutpumpe an, damit sich diese entgegen einer Therapierichtung dreht. Alternativ könnte die Blutpumpe jedoch auch eingerichtet sein, von einem okkludierten Zustand in einen nicht okkludierten Zustand zu wechseln (beispielsweise durch einziehbare Rollen). In diesem Fall müsste die Blutpumpe nicht zwangsläufig für einen Drehrichtungswechsel eingerichtet sein, um die arterielle Reinfusion durchführen zu können. Die arterielle Reinfusion könnte in diesem Fall somit auch ohne Drehbetätigung der Blutpumpe entgegen der Therapierichtung erfolgen.

Um das Blut dem Patienten sowohl über den arteriellen Abschnitt als auch über den venösen Abschnitt des extrakorporalen Kreislaufs zurückzugeben, wird die arterielle Blutpumpe bevorzugt jedoch mit einer konstanten Förderrate zur Erzeugung eines konstanten Flusses entgegen der Therapierichtung betrieben. Das bedeutet, dass die arterielle Flusspumpe bevorzugt eine Flüssigkeit von dem Dialysator in Richtung hin zu dem arteriellen Ende des extrakorporalen Kreislaufs fördert.

Wenn beispielsweise die Förderrate der arteriellen Flusspumpe derart eingestellt wird, dass ein Fluss in dem arteriellen Abschnitt entgegen der Therapierichtung erzeugt wird, welcher der Hälfte des über die Dialysatormembran übertretenden Flusses entspricht (beispielsweise 50 ml/min, wenn der definierte Fluss über die Dialysatormembran 100 ml/min ist), wird automatisch erreicht, dass der Fluss in dem venösen Abschnitt in Therapierichtung hin zu dem venösen Ende dem arteriellen Fluss entspricht. Denn der Fluss, welcher in den venösen Abschnitt eingebracht wird, ist die Differenz aus dem über die Dialysatormembran übertretenden Fluss und dem geförderten Fluss durch die arterielle Blutpumpe. Wenn schließlich die in dem extrakorporalen Kreislauf vorhandenen Schlauchklemmen (arterielle Schlauchklemme und venöse Schlauchklemme) geöffnet sind, kann das Blut sowohl in dem arteriellen Abschnitt als auch in dem venösen Abschnitt des extrakorporalen Kreislaufs durch die übertretende Dialysierflüssigkeit zum Patienten hin verdrängt werden.

Gemäß einem bevorzugten/ dem voranstehend beschriebenen Ausführungsbeispiel ist die Steuereinheit somit eingerichtet, die Reinfusion über den venösen Abschnitt und die Reinfusion über den arteriellen Abschnitt parallel/ simultan/ gleichzeitig durchzuführen.

Gemäß einem weiteren bzw. alternativen bevorzugten Ausführungsbeispiel kann die Steuereinheit jedoch auch eingerichtet sein, die Reinfusion über den venösen Abschnitt und die Reinfusion über den arteriellen Abschnitt seriell/ sukzedan/ nacheinander durchzuführen. Dabei kann grundsätzlich zunächst die Reinfusion über den venösen Abschnitt und anschließend die Reinfusion über den arteriellen Abschnitt durchgeführt werden. Alternativ kann grundsätzlich genauso zunächst die Reinfusion über den arteriellen Abschnitt und anschließend erst die Reinfusion über den venösen Abschnitt durchgeführt werden.

Beispielsweise kann eine serielle Blutrückgabe bzw. Reinfusion derart durchgeführt werden, dass zuerst das Blut aus der venösen Blutschlauchseite/ aus dem venösen Abschnitt an einen Patienten zurückgegeben wird, und anschließend erst das Blut aus der arteriellen Blutschlauchseite/ aus dem arteriellen Abschnitt reinfundiert wird. Dabei kann bevorzugt (wie bei der parallelen Reinfusion) nach einem Ende der Blutbehandlungstherapie die Reinfusion über einen Überdruck auf der Dialysierflüssigkeitsseite des Dialysators eingeleitet werden. Durch diesen Überdruck tritt die Dialysierflüssigkeit durch die Membran des Dialysators und dient als Reinfusionsflüssigkeit. Wenn nun die arterielle Blutpumpe angehalten ist und die venöse Schlauchklemme geöffnet ist, wird das Blut in dem venösen Schlauchsegment/ Abschnitt durch die übertretende Dialysierflüssigkeit zum Patienten hin verdrängt. Nach einem Abbruch der venösen Reinfusion, wechselt in vorteilhafter Weise die Reinfusionsrichtung. Insbesondere wird dann die venöse Schlauchklemme geschlossen und die arterielle Schlauchklemme wird geöffnet (wenn diese zuvor geschlossen war) bzw. bleibt geöffnet. Nun beginnt in vorteilhafter Weise die arterielle Blutpumpe entgegen der Therapierichtung zu drehen und gibt somit das Blut aus dem arteriellen Abschnitt Blutschlauchsystem an den Patienten zurück. In diesem Fall kann die Förderrate der arteriellen Blutpumpe auf den Fluss/ die Flussmenge der durch die Membran übertretenden Dialysierflüssigkeit eingestellt werden. Durch einen Abbruch der arteriellen Reinfusion wird der serielle Reinfusionsvorgang insgesamt beendet.

Alternativ kann eine serielle Blutrückgabe bzw. Reinfusion auch derart durchgeführt werden, dass zuerst das Blut aus der arteriellen Blutschlauchseite/ aus dem arteriellen Abschnitt an einen Patienten zurückgegeben wird, und anschließend erst das Blut aus der venösen Blutschlauchseite/ aus dem venösen Abschnitt reinfundiert wird. Dabei kann bevorzugt nach einem Ende der Blutbehandlungstherapie die Reinfusion über einen Überdruck auf der Dialysierflüssigkeitsseite des Dialysators eingeleitet werden. Durch diesen Überdruck tritt die Dialysierflüssigkeit durch die Membran des Dialysators und dient als Reinfusionsflüssigkeit. Wenn nun die venöse Schlauchklemme geschlossen wird bzw. geschlossen bleibt, die arterielle Schlauchklemme geöffnet wird bzw. geöffnet bleibt und die arterielle Blutpumpe beginnt, entgegen der Therapierichtung zu drehen, wird das Blut aus dem arteriellen Abschnitt Blutschlauchsystem an den Patienten zurückgegeben. Die Förderrate der arteriellen Blutpumpe kann dabei auf den Fluss/ die Flussmenge der durch die Membran übertretenden Dialysierflüssigkeit eingestellt werden. Nach einem Abbruch der arteriellen Reinfusion (beispielsweise durch ein Anhalten der arteriellen Blutpumpe/ ein Schließen der arteriellen Schlauchklemme), wechselt in vorteilhafter Weise die Reinfusionsrichtung. Insbesondere wird dann die venöse Schlauchklemme geöffnet, und das Blut wird in dem venösen Schlauchsegment/ Abschnitt durch die übertretende Dialysierflüssigkeit zum Patienten hin verdrängt. Durch einen Abbruch der venösen Reinfusion wird der serielle Reinfusionsvorgang insgesamt beendet.

In anderen Worten erfolgt die serielle Blutrückgabe "arteriell-venös" analog zu der seriellen Blutrückgabe "venös-arteriell". Die Maschinenkomponenten werden lediglich derart gesteuert, dass zuerst der arterielle Abschnitt geleert wird und dann erst der venöse Abschnitt geleert wird.

Es ist von Vorteil, wenn die Steuereinheit eingerichtet ist, die Reinfusion in Abhängigkeit von dem verwendeten Dialysator zu steuern.

Insbesondere kann die Steuereinheit dabei eingerichtet sein, den verwendeten Dialysator in Abhängigkeit von dem Ultrafiltrationskoeffizienten (KUF) desselben als High-Flux-Dialysator oder als Low-Flux-Dialysator zu klassifizieren, und auf der Grundlage der Klassifizierung eine geeignete Steuerung (der Flusspumpe-Eingang), insbesondere eine High-Flux-Steuerung oder eine Low-Flux-Steuerung, durchzuführen.

Grundsätzlich gilt, dass eine Membran von Low-Flux-Dialysatoren kleine Poren aufweist, und dass eine Membran von High-Flux-Dialysatoren große Poren aufweist. Es versteht sich somit von selbst, dass eine Flüssigkeit eine Membran von High-Flux-Dialysatoren einfacher/ besser passieren kann als eine Membran von Low-Flux-Dialysatoren. Um einen gewünschten Fluss (Flussrate/ Volumenstrom) zu erreichen muss vor diesem Hintergrund zwangsläufig der Druck bei Low-Flux-Dialysatoren größer eingestellt werden als bei High-Flux-Dialysatoren.

In vorteilhafter Weise wird somit offenbarungsgemäß die Steuerung der Reinfusion an den verwendeten Dialysator angepasst.

Dabei kann der Ultrafiltrationskoeffizient der Steuereinheit bereits bekannt sein, beispielsweise, wenn Informationen über den verwendeten Dialysator (umfassend den Ultrafiltrationskoeffizienten) von einem Lesegerät vor der Blutbehandlungstherapie eingelesen werden und der Steuereinheit übermittelt werden bzw. die Informationen von einem Anwender vor der Blutbehandlungstherapie manuell eingegeben werden, oder kann die Steuereinheit den Ultrafiltrationskoeffizienten während der Blutbehandlungstherapie ermitteln. Beispielsweise kann während der Blutbehandlungstherapie ein konstanter Ultrafiltrationsfluss QUF (beispielsweise 70 ml/min) eingestellt werden und der Transmembrandruck nach einer kurzen Einstellzeit (beispielsweise 40 Sekunden) bestimmt werden. Aus diesen Informationen kann in bekannter Weise der Ultrafiltrationskoeffizient (KUF) berechnet werden.

Bevorzugt wird die High-Flux-Steuerung durchgeführt, wenn der Ultrafiltrationskoeffizient größer einem vorbestimmten Wert, welcher beispielsweise bei 20 ml/(min*mmHg) liegt, ist, und wird die Low-Flux-Steuerung durchgeführt, wenn der Ultrafiltrationskoeffizient kleiner dem vorbestimmten Wert ist. In vorteilhafter Weise kann somit auch bei Dialysatoren von Fremdherstellern offenbarungsgemäß der Ultrafiltrationskoeffizient des entsprechenden Dialysators bestimmt werden, und eine Klassifizierung als High-Flux-Dialysator oder als Low-Flux-Dialysator vorgenommen werden, um die Reinfusion in Abhängigkeit davon steuern zu können.

Bevorzugt ist die Steuereinheit eingerichtet, nach der Blutbehandlungstherapie und vor der Reinfusion einen (Sicherheits-) Luftentfernungsschritt durchzuführen, mittels welchem Luft bzw. eine Luftblase aus dem arteriellen Abschnitt entfernt wird.

In vorteilhafter Weise sind in dem arteriellen Abschnitt eine arterielle Schlauchklemme und eine/die arterielle Blutpumpe vorgesehen, und ist in dem venösen Abschnitt eine venöse Expansionskammer/ Luftfalle vorgesehen, und führt die Steuereinheit den (Sicherheits-) Luftentfernungsschritt derart durch, dass zunächst der arterielle Abschnitt für eine vorbestimmte Zeitdauer (beispielsweise zwei Sekunden) über die arterielle Schlauchklemme abgeklemmt wird, während die arterielle Blutpumpe in Therapierichtung weiterläuft, wobei der dadurch entstehende Unterdruck die in dem arteriellen Abschnitt vorhandene Luft bzw. die Luftblase mitreißt und in den venösen Abschnitt befördert, wo sie in der venösen Expansionskammer/ Luftfalle eliminiert wird.

Offenbarungsgemäß hat sich herausgestellt, dass sich bei der Blutbehandlungstherapie/ der Dialysebehandlung, insbesondere wenn der extrakorporale Blutkreislauf und der Dialysator mit Blut befüllt werden, an einem Eingang der arteriellen Blutpumpe eine Luftblase befindet. Grundsätzlich können sich jedoch auch an anderen Abschnitten/ Orten in dem arteriellen Abschnitt des extrakorporalen Kreislaufs Luft oder Luftblasen befinden. Bei der arteriellen Reinfusion, insbesondere bei einem Betreiben der arteriellen Blutpumpe entgegen der Therapierichtung, besteht dabei grundsätzlich die Gefahr, dass eine sich in dem arteriellen Abschnitt befindliche Luftblase nicht mehr aus dem extrakorporalen Kreislauf entfernt werden kann (beispielsweise, weil keine arterielle Expansionskammer! Luftfalle zum Entfernen von Luftblasen vorgesehen ist) und somit in den Patienten gelangen kann. Dies muss unbedingt vermieden werden, da ein Luftbolus schädlich für den Patienten ist.

Wenn dieser Verfahrensschritt zur Sicherheit (Sicherheits-Luftentfernungsschritt) von der Steuereinheit vor der Reinfusion durchgeführt wird, kann eine einwandfreie arterielle Blutrückgabe gewährleistet werden.

In vorteilhafter Weise liegt der vorbestimmte Grenzwert für den Hämatokrit-Anteil bei kleiner oder gleich 10%, bevorzugt zwischen 2% und 5%, besonders bevorzugt bei 3%. Beispielsweise wird offenbarungsgemäß der vorbestimmte Grenzwert auf einen Hämatokrit-Anteil von 5% oder einen Hämatokrit-Anteil von 3% festgelegt, so dass der vorbestimmte Grenzwert unterschritten wird, wenn der Hämatokrit-Anteil kleiner oder gleich 5%/ kleiner oder gleich 3% ist.

Insbesondere hat sich offenbarungsgemäß herausgestellt, dass wenn der vorbestimmte Grenzwert für den Hämatokrit-Anteil auf einen prozentualen Wert eingestellt wird, welcher in dem genannten Bereich liegt, ein Abbruch der Reinfusion nicht zu früh vorgenommen wird, das heißt noch nicht vorgenommen wird, wenn sich noch zu viel Blut des Patienten in dem extrakorporalen Kreislauf befindet. Dabei sollte der vorbestimmte Grenzwert bevorzugt auch nicht auf einen zu kleinen Hämatokrit-Anteil/ prozentualen Wert, beispielsweise weniger als 2%, eingestellt werden, da in diesem Fall dem Patienten bereits übermäßig viel Dialysierflüssigkeit/ Reinfusionsflüssigkeit zugeführt wird. Besonders bevorzugt ist es offenbarungsgemäß, wenn der vorbestimmte Grenzwert auf 3% festgelegt wird. Wenn offenbarungsgemäß von einem Grenzwert von 3% gesprochen wird, ist selbstverständlich nicht "genau 3%" sondern "näherungsweise/ etwa 3%" zu verstehen.

Gemäß einem bevorzugten Ausführungsbeispiel ist der erste Sensor ein venöser Sicherheitsluftdetektor mit integriertem Rotdetektor, welcher eingerichtet ist, Luft oder Luftblasen in der den venösen Abschnitt durchströmenden Flüssigkeit zu erkennen und den Hämatokrit-Anteil der Flüssigkeit als Abbruchkriterium der Reinfusion über den venösen Abschnitt zu erfassen, und ist der zweite Sensor ein arterieller Sicherheitsluftdetektor mit integriertem Rotdetektor, welcher eingerichtet ist, Luft oder Luftblasen in der den arteriellen Abschnitt durchströmenden Flüssigkeit zu erkennen und den Hämatokrit-Anteil der Flüssigkeit als Abbruchkriterium der Reinfusion über den arteriellen Abschnitt zu erfassen.

In anderen Worten zeichnet sich ein Ausführungsbeispiel bevorzugt dadurch aus, dass sowohl auf der arteriellen Seite/ in dem arteriellen Abschnitt als auch auf der venösen Seite/ in dem venösen Abschnitt ein Sicherheitsluftdetektor mit integriertem Rotdetektor vorgesehen ist bzw. bereitgestellt wird. Somit sind bevorzugt zwei Sicherheitsluftdetektoren mit integrierten Rotdetektoren in der extrakorporalen Blutbehandlungsvorrichtung vorgesehen, welche Luft bzw. Luftblasen zuverlässig erkennen und auch einen Hämatokrit-Grenzwert als Abbruchkriterium für die Reinfusion erfassen können.

Da gemäß diesem Ausführungsbeispiel Luft bzw. Luftblasen verlässlich sowohl in dem arteriellen Abschnitt als auch in dem venösen Abschnitt detektiert werden können (durch das Bereitstellen von zwei Sicherheitsluftdetektoren), muss der oben beschriebene (Sicherheits-) Luftentfernungsschritt nicht zwangsläufig durchgeführt werden. Denn wenn sich Luft bzw. Luftblasen in dem arteriellen Abschnitt befinden würden, würden auch diese - sofern sie sich lösen und in Richtung Patient gefördert werden - durch den vorgesehenen Sicherheitsluftdetektor erfasst, und die Steuereinheit könnte in einem solchen Fall grundsätzlich die Reinfusion durch Stoppen der arteriellen Blutpumpe oder durch Schließen der arteriellen Schlauchklemme oder der venösen Schlauchklemme abbrechen.

Da sich gemäß diesem Ausführungsbeispiel sowohl in dem arteriellen Abschnitt als auch in dem venösen Abschnitt ein Sicherheitsluftdetektor mit integriertem Rotdetektor befindet (sogenannter SADRDV-Sensor), kann - insbesondere wenn ein Gemisch aus Blut und Dialysierflüssigkeit durch den arteriellen Abschnitt/ den venösen Abschnitt das entsprechende Schlauchsegment strömt - ein Rot-Wert/ eine Farbmaßzahl des Blut-Dialysierflüssigkeit-Gemisches in dem entsprechenden Schlauchsegment bestimmt werden. Aus dem erfassten Rot-Wert kann mit dem verwendeten Sicherheitsluftdetektor mit integriertem Rotdetektor recht genau ein Hämatokrit-Anteil des Blut-Dialysierflüssigkeits-Gemisches bestimmt werden. Dies gilt auch, wenn der Hämatokrit-Anteil bereits sehr gering ist. Sobald die Rotdetektoren der SADRDV-Sensoren einen Hämatokrit-Anteil unter dem angesprochenen vorbestimmten Grenzwert erfassen (beispielsweise kleiner 3%), wird die Reinfusion in dem entsprechenden Schlauchsystem, in welchem erfasst wurde, dass der vorbestimmte Grenzwert unterschritten wird, beendet.

Dabei kann es grundsätzlich vorkommen, dass das Blut in einem Abschnitt des extrakorporalen Kreislaufs/ in einem Segment des Blutschlauchsystems (z.B. in dem arteriellen Abschnitt) schneller zurückgeführt wird, als das Blut in dem anderen Abschnitt des extrakorporalen Kreislaufs/ dem anderen Segment des Blutschlauchsystems (z.B. in dem venösen Abschnitt). In diesem Fall ist die Steuereinheit eingerichtet, die Fördermenge/ den Fluss der über die Membran des Dialysators tretenden Dialysierflüssigkeit beizubehalten oder zu verringern, und nun vollständig über den Abschnitt des extrakorporalen Kreislaufs zu leiten, in welchem die Reinfusion noch nicht abgebrochen wurde.

Wenn die Blutreinfusion über den venösen Abschnitt des extrakorporalen Kreislaufs früher abgebrochen wird, schließt die Steuereinheit beispielsweise die venöse Schlauchklemme und steuert die arterielle Blutpumpe an, damit diese mit einer Förderrate der übertretenden Dialysierflüssigkeit entgegen der Therapierichtung betrieben wird. Die übertretende Dialysierflüssigkeit/ Reinfusionsflüssigkeit verdrängt dadurch das restliche Blut in den arteriellen Teil des Blutschlauchsystems, bis der dort vorgesehene SADRDV-Sensor den festgelegten Hämatokrit-Grenzwert detektiert.

Bei einem früheren Abbruch der Blutreinfusion über den arteriellen Abschnitt des extrakorporalen Kreislaufs, wird die arterielle Blutpumpe gestoppt und die arterielle Schlauchklemme geschlossen. Dann verdrängt die übertretende Reinfusionsflüssigkeit/ Dialysierflüssigkeit das restliche Blut in den venösen Abschnitt des extrakorporalen Kreislaufs, bis der dort vorgesehene SADRDV-Sensor den festgelegten Hämatokrit-Grenzwert detektiert.

Die Luftdetektoren der SADRDV-Sensoren überwachen dabei kontinuierlich, dass kein Lufteintritt in den Patienten stattfindet. Sollte eine Luftblase detektiert werden, stoppt die Steuereinheit das Reinfusionsverfahren. Insbesondere schließt die Steuereinheit dann die arterielle Schlauchklemme und die venöse Schlauchklemme, hält die Blutpumpe an, und hebt den Überdruck auf der Dialysierflüssigkeitsseite des Dialysators auf (beispielsweise durch Stoppen der Flusspumpe-Eingang und Öffnen des Dialysatorauslassventils).

Das voranstehend beschriebene Reinfusionsverfahren kann analog angewendet werden, wenn die Reinfusion nicht parallel, sondern seriell durchgeführt wird. Wenn bei der seriellen Reinfusion beispielsweise zunächst über den venösen Abschnitt des extrakorporalen Kreislaufs Blut dem Patienten zurückgegeben wird, erfasst/ überwacht der Rotdetektor des SADRDV-Sensors den Hämatokrit-Anteil des Blutes bzw. des Blut-Dialysierflüssigkeits-Gemisches. Wenn der Rotdetektor dabei einen Hämatokrit-Anteil unter dem vorbestimmten Grenzwert (beispielsweise kleiner 3%) erkennt, wird die Reinfusion über den venösen Abschnitt abgebrochen bzw. beendet. Es folgt in diesem beispielhaften Fall die Reinfusion über den arteriellen Abschnitt. Der in dem arteriellen Abschnitt vorgesehene Rotdetektor des SADRDV-Sensors erfasst/ überwacht den Hämatokrit-Anteil des Blutes bzw. des Blut-Dialysierflüssigkeits-Gemisches in dem arteriellen Abschnitt. Wenn der Rotdetektor dabei einen Hämatokrit-Anteil unter dem vorbestimmten Grenzwert (beispielsweise kleiner 3%) erkennt, wird auch die Reinfusion über den arteriellen Abschnitt abgebrochen bzw. beendet.

Gemäß einem weiteren bzw. alternativen bevorzugten Ausführungsbeispiel ist der erste Sensor ein venöser Sicherheitsluftdetektor mit integriertem Rotdetektor, welcher eingerichtet ist, Luft oder Luftblasen in der den venösen Abschnitt durchströmenden Flüssigkeit zu erkennen und den Hämatokrit-Anteil der Flüssigkeit als Abbruchkriterium der Reinfusion über den venösen Abschnitt zu erfassen, und ist der zweite Sensor ein Hämatokrit-Sensor (HTC-Sensor), welcher eingerichtet ist, Informationen über den Hämatokrit-Anteil in der Flüssigkeit während der Reinfusion zu erfassen und diese Informationen an die Steuereinheit weiterzugeben.

In anderen Worten kann grundsätzlich in dem arteriellen Abschnitt des extrakorporalen Blutkreislaufs anstelle des Sicherheitsluftdetektors mit integriertem Rotdetektor (SADRDV-Sensor) ein Hämatokrit-Sensor bzw. HTC-Sensor zur Erfassung eines Hämatokrit-Anteils verwendet werden. Insbesondere ist bei vielen extrakorporalen Blutbehandlungsvorrichtungen/ Dialysemaschinen in dem arteriellen Abschnitt des extrakorporalen Kreislaufs standardmäßig bereits ein Hämatokrit-Sensor (HTC-Sensor) vorgesehen. Dieses Ausführungsbeispiel hat somit den Vorteil, dass an der arteriellen Seite/ in dem arteriellen Abschnitt kein zusätzlicher Sicherheitsluftdetektor integriert werden muss (Hardware-Änderung), sondern dass bei vielen Maschinen lediglich eine Software-Änderung vorgenommen werden muss.

Da ein HTC-Sensor keinen Luftdetektor beinhaltet, muss bei diesem Ausführungsbeispiel in geeigneter Weise sichergestellt werden, dass keine Luft in den Patienten eintritt. Um zu vermeiden, dass ein zusätzlicher Luftdetektor in den arteriellen Abschnitt des extrakorporalen Kreislaufs integriert werden muss, ist bei diesem Ausführungsbeispiel der oben beschriebene (Sicherheits-) Luftentfernungsschritt unabdingbar bzw. zwingend erforderlich.

Es hat sich insbesondere offenbarungsgemäß herausgestellt, dass der oben beschriebene Sicherheits-Luftentfernungsschritt es ermöglicht, dass auf einen Luftdetektor in dem arteriellen Abschnitt des extrakorporalen Kreislaufs verzichtet werden kann, und somit in vorteilhafter Weise in dem arteriellen Abschnitt auch lediglich ein kostengünstiger, in vielen Dialysemaschinen bereits vorhandener Hämatokrit-Sensor verwendet werden kann.

Problematisch ist an dieser Stelle jedoch, dass lediglich ein Luftdetektor mit integriertem Rotdetektor (SADRDV-Sensor) einen Hämatokrit-Anteil bei geringen prozentualen Werten verlässlich messen kann. Hämatokrit-Sensoren (HTC-Sensoren) können einen Hämatokrit-Wert/Anteil lediglich in einem Bereich zwischen 20% und 55% zuverlässig messen, mit Abstrichen in einem Bereich zwischen 10% und 55%. Da der vorbestimmte Grenzwert offenbarungsgemäß jedoch bei kleiner 10% liegt, kann der Hämatokrit-Sensor nicht zuverlässig erfassen, wann der vorbestimmte Grenzwert der vorliegenden Offenbarung unterschritten wird. Vor diesem Hintergrund ist es - insbesondere, wenn ein Hämatokrit-Sensor verwendet wird - erforderlich, dass die Steuereinheit auf der Grundlage von von dem Hämatokrit-Sensor erfassten Informationen berechnet oder vorhersagt, dass/ wann der Hämatokrit-Anteil den vorbestimmten Grenzwert unterschreitet.

Bevorzugt ist die Steuereinheit daher eingerichtet, auf der Grundlage der Informationen über den Hämatokrit-Anteil in der Flüssigkeit eine Berechnung oder eine Vorhersage dahingehend vorzunehmen, wann der Hämatokrit-Anteil der Flüssigkeit den vorbestimmten Grenzwert unterschreitet. In anderen Worten verwendet die Steuereinheit bevorzugt die ihr von dem Hämatokritsensor übermittelten Informationen, insbesondere Informationen über den Hämatokrit-Anteil in dem prozentualen Bereich, in welchem dieser zuverlässig misst, um zu berechnen bzw. vorherzusagen, wann mit einem Unterschreiten des vorbestimmten Grenzwerts zu rechnen ist, und bricht den Reinfusionsvorgang auf der Grundlage dieser Berechnung/ Vorhersage ab.

Weiter bevorzugt ist die Steuereinheit eingerichtet, die Berechnung oder die Vorhersage mittels einer linearen Funktion vorzunehmen, wenn sie die Reinfusion über den venösen Abschnitt und die Reinfusion über den arteriellen Abschnitt seriell/ sukzedan/ nacheinander durchführt oder wenn ein Reinfusionsfluss in dem arteriellen Abschnitt oder dem venösen Abschnitt groß ist bzw. größer einem vorbestimmten Schwellwert (z.B. 75 ml/min) ist.

Weiter bevorzugt ist die Steuereinheit eingerichtet, die Berechnung oder die Vorhersage mittels einer Gauß-Funktion (erster Ordnung) vorzunehmen, wenn sie die Reinfusion über den venösen Abschnitt und die Reinfusion über den arteriellen Abschnitt parallel/ simultan/ gleichzeitig durchführt oder wenn ein Reinfusionsfluss in dem arteriellen Abschnitt oder dem venösen Abschnitt klein ist bzw. kleiner einem vorbestimmten Schwellwert (z.B. 75 ml/min) ist.

In anderen Worten ist die Steuereinheit bevorzugt eingerichtet, die Berechnung oder die Vorhersage mittels einer linearen Funktion vorzunehmen, wenn der Reinfusionsfluss über einem vorbestimmten Grenzwert/ Schwellwert ist, und die Berechnung oder die Vorhersage mittels einer Gauß-Funktion vorzunehmen, wenn der Reinfusionsfluss unter einem vorbestimmten Grenzwert/ Schwellwert ist. Es wird somit eine lineare Funktion zur Vorhersage verwendet, wenn der Reinfusionsfluss groß/ hoch ist und es somit zu weniger Vermischungsvorgängen zwischen Blut und Dialysierflüssigkeit bei der Reinfusion kommt, und es wird eine Gauß-Funktion zur Vorhersage verwendet, wenn der Reinfusionsfluss klein/ gering ist und es somit zu mehr Vermischungsvorgängen zwischen Blut und Dialysierflüssigkeit bei der Reinfusion kommt.

Die Steuereinheit ist somit eingerichtet, wenn ein Hämatokritsensor (HTC-Sensor) zum Messen des Hämatokrit-Anteils verwendet wird, die Messergebnisse des Hämatokritsensors intelligent auszuwerten.

Zusammengefasst betrifft die Offenbarung somit eine extrakorporale Blutbehandlungsvorrichtung mit: einem extrakorporalen Kreislauf, einem Dialysator, einem Dialysierflüssigkeitskreislauf und einer Steuereinheit. Sowohl in einem venösen Abschnitt als auch in einem arteriellen Abschnitt des extrakorporalen Kreislaufs ist ein Sensor vorgesehen, welcher eingerichtet ist, einen Hämatokrit-Anteil in einer den entsprechenden Abschnitt durchströmenden Flüssigkeit zu erfassen. Die Steuereinheit ist eingerichtet, eine Reinfusion von Blut derart zu steuern, dass eine Dialysierflüssigkeit von dem Dialysierflüssigkeitskreislauf über eine Dialysatormembran an den extrakorporalen Kreislauf geliefert wird, welche bei der Reinfusion das in dem extrakorporalen Kreislauf vorhandene Blut zu einem Patienten hin verdrängt, um das Blut dem Patienten über den venösen Abschnitt und den arteriellen Abschnitt zurückzugeben. Dabei wird die Reinfusion in dem entsprechenden Abschnitt des extrakorporalen Kreislaufs sensorgesteuert abgebrochen, wenn von dem entsprechenden Sensor erfasst wird oder von der Steuereinheit auf der Grundlage von von dem entsprechenden Sensor erfassten Informationen berechnet oder vorhergesagt wird, dass der Hämatokrit-Anteil einen vorbestimmten Grenzwert unterschreitet.

Die vorliegende Offenbarung stellt insgesamt eine automatische und direkte Einleitung der Reinfusion nach der Dialysebehandlung bereit. Die offenbarungsgemäße Reinfusion spart Materialkosten, da zur Reinfusion kein Kochsalzbeutel verwendet wird. Außerdem ist die Reinfusion auch in Maschinen ohne Substituatport anwendbar. Die Offenbarung ermöglicht, dass das Pflegepersonal zwischen dem Ende der Dialysebehandlung und dem Beginn der Reinfusion nicht aktiv eingreifen muss. Insbesondere ist keine Interaktion mit dem Pflegepersonal zum Abkoppeln eines Patientenanschlusses vonnöten. Weiterhin stellt die Offenbarung ein verbessertes Abbrechen der Reinfusion bereit, wenn die Reinfusion sowohl über den arteriellen Abschnitt als auch über den venösen Abschnitt des extrakorporalen Kreislaufs vorgenommen wird. Darüber hinaus wird sichergestellt, dass es zu keinem Lufteintritt in den Patienten kommt.

Weiterhin betrifft die vorliegende Offenbarung ein Verfahren zum automatischen Durchführen einer Reinfusion von Blut nach einer Blutbehandlungstherapie mit den Schritten: Liefern einer Dialysierflüssigkeit von einem Dialysierflüssigkeitskreislauf über eine Membran eines Dialysators an einen extrakorporalen Kreislauf; Verdrängen des in dem extrakorporalen Kreislauf vorhandenen Bluts hin zu einem Patienten; Zurückgeben des Bluts an den Patienten sowohl über einen venösen Abschnitt des extrakorporalen Kreislaufs als auch über einen arteriellen Abschnitt des extrakorporalen Kreislaufs; Abbrechen der Reinfusion über den venösen Abschnitt, wenn von einem ersten in dem venösen Abschnitt vorgesehenen Sensor erfasst wird oder wenn auf der Grundlage von von dem ersten Sensor erfassten Informationen vorhergesagt oder berechnet wird, dass ein Hämatokrit-Anteil einer durch den venösen Abschnitt strömenden, Blut enthaltenden Flüssigkeit einen vorbestimmten Grenzwert unterschreitet; und Abbrechen der Reinfusion über den arteriellen Abschnitt, wenn von einem zweiten in dem arteriellen Abschnitt vorgesehenen Sensor erfasst wird oder wenn auf der Grundlage von von dem zweiten Sensor erfassten Informationen vorhergesagt oder berechnet wird, dass ein Hämatokrit-Anteil einer durch den arteriellen Abschnitt strömenden, Blut enthaltenden Flüssigkeit einen vorbestimmten Grenzwert unterschreitet.

Um das offenbarungsgemäße Verfahren durchführen zu können, wird bevorzugt eine antegrade Punktion (in Flussrichtung) der arteriellen Nadel im Patienten vorgenommen, da bei einer retrograden Punktion (gegen die Flussrichtung) der arteriellen Nadel Turbulenzen im Gefäß entstehen können.

Nachdem das offenbarungsgemäße Verfahren durchgeführt wurde bzw. die Reinfusion sowohl über den arteriellen Abschnitt als auch über den venösen Abschnitt abgebrochen wurde und die arterielle Schlauchklemme und die venöse Schlauchklemme geschlossen wurden, kann der Patient von einem Pflegepersonal sowohl arteriell als auch venös abgekoppelt werden. In vorteilhafter Weise werden anschließend die patientenseitigen Anschlüsse miteinander verbunden bzw. kurzgeschlossen. In anderen Worten wird bevorzugt das Ende des arteriellen Abschnitts mit dem Ende des venösen Abschnitts verbunden. In vorteilhafter Weise wird anschließend das Verfahren durchgeführt, welches in der EP 3 231 466 B1 beschrieben ist. Insbesondere stellt die EP 3 231 466 B1 ein geeignetes Verfahren zum Leeren des Blutschlauchsystems und des blutseitigen Dialysators bereit, nachdem der Patient abgekoppelt wurde.

## Kurzbeschreibung der Figuren

Die Offenbarung wird nachfolgend mit Hilfe von Figuren weiter erläutert. Es zeigen:

Fig. 1    eine extrakorporale Blutbehandlungsvorrichtung gemäß einem ersten bevorzugten Ausführungsbeispiel der vorliegenden Offenbarung, anhand derer die offenbarungsgemäße automatische Reinfusion erläutert wird;

Fig. 2    eine extrakorporale Blutbehandlungsvorrichtung gemäß einem zweiten bevorzugten Ausführungsbeispiel der vorliegenden Offenbarung, anhand derer die offenbarungsgemäße automatische Reinfusion erläutert wird;

Fig. 3    eine Ansicht einer arteriellen Blutpumpe, bevor ein offenbarungsgemäßer Sicherheits-Luftentfernungsschritt durchgeführt wird;

Fig. 4    eine Ansicht der arteriellen Blutpumpe, nachdem der offenbarungsgemäße Sicherheits-Luftentfernungsschritt durchgeführt wurde;

Fig. 5    ein Diagramm, welches den Verlauf eines einem Patienten zugeführten Luftvolumenstroms bei der Reinfusion über den arteriellen Abschnitt des extrakorporalen Kreislaufs über die Zeit darstellt, nachdem der offenbarungsgemäße Sicherheits-Luftentfernungsschritt durchgeführt wurde;

Fig. 6    ein Diagramm, welches eine Vorhersage eines Hämatokrit-Anteils mittels einer linearen Funktion veranschaulichen soll, wenn die Reinfusion über den arteriellen Abschnitt und den venösen Abschnitt seriell durchgeführt wird;

Fig. 7    ein Diagramm, welches eine Vorhersage eines Hämatokrit-Anteils mittels einer linearen Funktion veranschaulichen soll, wenn die Reinfusion über den arteriellen Abschnitt und den venösen Abschnitt parallel durchgeführt wird; und

Fig. 8    ein Diagramm, welches eine Vorhersage eines Hämatokrit-Anteils mittels einer Gauß-Funktion veranschaulichen soll, wenn die Reinfusion über den arteriellen Abschnitt und den venösen Abschnitt parallel durchgeführt wird.

## Beschreibung der Ausführungsbeispiele

Die Figuren sind lediglich schematischer Natur und dienen ausschließlich dem Verständnis der Offenbarung. Gleiche Elemente sind dabei mit denselben Bezugszeichen versehen. Die Merkmale der einzelnen Ausführungsbeispiele können untereinander ausgetauscht werden, wenn dies nicht explizit anders beschrieben wird.

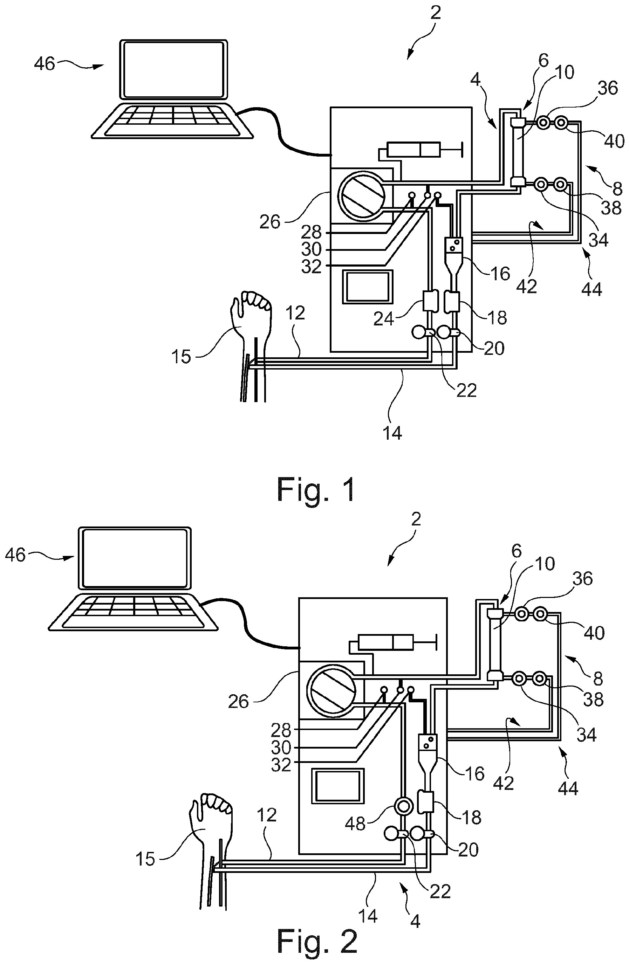

Fig. 1 zeigt eine extrakorporale Blutbehandlungsvorrichtung (Dialysemaschine) 2 gemäß einem ersten bevorzugten Ausführungsbeispiel der vorliegenden Offenbarung, anhand derer die offenbarungsgemäße automatische Reinfusion erläutert wird.

Die extrakorporale Blutbehandlungsvorrichtung 2 enthält grundsätzlich einen extrakorporalen Kreislauf (AN-Schlauchsystem) 4, einen Dialysator 6 und einen Dialysierflüssigkeitskreislauf 8. Dabei sind der extrakorporale Kreislauf 4 und der Dialysierflüssigkeitskreislauf 8 über eine in dem Dialysator 6 vorgesehene Membran 10 voneinander getrennt.

Der extrakorporale Kreislauf 4 enthält einen arteriellen Abschnitt 12, welcher sich stromaufwärts des Dialysators 6 befindet, und einen venösen Abschnitt 14, welcher sich stromabwärts des Dialysators 6 befindet.

Wie aus Fig. 1 ersichtlich ist, sind der arterielle Abschnitt 12 und der venöse Abschnitt 14 an einem Patienten 15 angekoppelt. In anderen Worten ist ein Ende des arteriellen Abschnitts 12 an eine Arterie des Patienten 15 angekoppelt und ist ein Ende des venösen Abschnitts 14 an eine Vene des Patienten 15 angekoppelt.

In dem venösen Abschnitt 14 des extrakorporalen Kreislaufs 4 sind stromabwärts des Dialysators 6 (das heißt ausgehend von dem Dialysator 6 in einer Richtung hin zu dem Ende des venösen Abschnitts 14) eine venöse Expansionskammer bzw. Luftfalle 16, ein venöser Sicherheitsluftdetektor mit integriertem Rotdetektor 18 und eine venöse Schlauchklemme 20 vorgesehen.

In dem arteriellen Abschnitt 12 sind ausgehend von dem patientenseitigen Ende des arteriellen Abschnitts 12 in einer Richtung hin zu dem Dialysator 6 eine arterielle Schlauchklemme 22, ein arterieller Sicherheitsluftdetektor mit integriertem Rotdetektor 24 und eine (arterielle) Blutpumpe 26 vorgesehen. Wie in Fig. 1 ersichtlich ist, ist der extrakorporale Kreislauf 4 (insbesondere ein Blutpumpenadapter desselben) bereits in die Blutpumpe 26, welche bevorzugt als Rollenpumpe bzw. Peristaltikpumpe ausgebildet ist und eingerichtet ist, durch Quetschen eines Schlauchs ein Fluid/ eine Flüssigkeit zu fördern, eingesetzt.

In dem arteriellen Abschnitt 12 kann ein arterieller Druck stromaufwärts bzw. vor der Blutpumpe 26 mit einem arteriellen Drucksensor 28 gemessen werden. Weiterhin kann ein Dialysatoreingangsdruck stromabwärts bzw. nach der Blutpumpe 26 und stromaufwärts bzw. vor dem Dialysator 6 (zwischen Dialysator 6 und Blutpumpe 26) über einen Dialysatoreingangsdrucksensor 30 gemessen werden. In dem venösen Abschnitt 14 kann ein venöser Druck an/ hinter der venösen Expansionskammer bzw. Luftfalle 16 über einen venösen Drucksensor 32 gemessen werden. Die in dem extrakorporalen Kreislauf 4 vorgesehenen Drucksensoren 28, 30, 32 können den Druck an den entsprechenden Stellen in dem extrakorporalen Kreislauf 4, an welchen diese angeordnet vorgesehen sind, messen/ abnehmen/ überwachen.

Der Dialysierflüssigkeitskreislauf 8 enthält ein Dialysatoreinlassventil 34, ein Dialysatorauslassventil 36, eine Flusspumpe-Eingang 38 und eine Flusspumpe-Ausgang 40. Grundsätzlich ist es jedoch ausreichend, wenn lediglich eine Flusspumpe, beispielsweise die Flusspumpe-Eingang 38, in dem Dialysierflüssigkeitskreislauf 8 vorgesehen ist. Das Dialysatoreinlassventil 34 und die Flusspumpe-Eingang 38 sind dabei an einem Dialysierflüssigkeitszufluss 42 stromaufwärts des Dialysators 6 vorgesehen/ angeordnet. Das Dialysatorauslassventil 36 und die Flusspumpe-Ausgang 40 sind an einem Dialysierflüssigkeitsabfluss 44 stromabwärts des Dialysators 6 vorgesehen/ angeordnet. Die Flusspumpe-Eingang 38 und die Flusspumpe-Ausgang 40 sind bevorzugt Zahnradpumpen.

Die extrakorporale Blutbehandlungsvorrichtung 2 weist weiterhin eine Steuereinheit 46 auf, welche bevorzugt als ein Prozessor, insbesondere als eine zentrale Rechen-/ bzw. Verarbeitungseinheit (CPU), ausgebildet ist. Die Steuereinheit 46 erhält Informationen von Sensoren, welche in der extrakorporalen Blutbehandlungsvorrichtung 2 vorgesehen sind. Lediglich beispielhaft sind dabei die in Fig. 1 gezeigten Sensoren zu nennen, also der arterielle Drucksensor 28, der Dialysatoreingangsdrucksensor 30, der venöse Drucksensor 32, der arterielle Sicherheitsluftdetektor mit integriertem Rotdetektor 24, der venöse Sicherheitsluftdetektor mit integriertem Rotdetektor 18, etc.. Auf der anderen Seite steuert bzw. betätigt die Steuereinheit 46 Aktoren, welche in der extrakorporalen Blutbehandlungsvorrichtung 2 vorgesehen sind. Lediglich beispielhaft sind dabei die in Fig. 1 gezeigten Ventile, Pumpen, Schlauchklemmen, etc., also insbesondere das Dialysatoreinlassventil 34, das Dialysatorauslassventil 36, die Flusspumpe-Eingang 38, die Flusspumpe-Ausgang 40, die (arterielle) Blutpumpe 26, die arterielle Schlauchklemme 22, die venöse Schlauchklemme 20, etc. zu nennen.

Der in Fig. 2 gezeigte Aufbau einer extrakorporalen Blutbehandlungsvorrichtung (Dialysemaschine) 2 (zweites bevorzugtes Ausführungsbeispiel der vorliegenden Offenbarung) unterscheidet sich von dem in Fig. 1 gezeigten Aufbau hardwaretechnisch lediglich darin, dass kein arterieller Sicherheitsluftdetektor mit integriertem Rotdetektor 24 vorgesehen ist, sondern stattdessen ein Hämatokritsensor (HTC-Sensor) 48 in dem arteriellen Abschnitt 12 des extrakorporalen Kreislaufs 4 angeordnet ist. Im Übrigen ist der in Fig. 2 gezeigte Aufbau identisch zu dem in Fig. 1 gezeigten Aufbau, so dass die voranstehenden Ausführungen bzw. Beschreibungen mutatis mutandis für den in Fig. 2 gezeigten Aufbau gelten und daher nicht wiederholt werden.

Fig. 1 und Fig. 2 zeigen jeweils einen Zustand unmittelbar nach Abschluss einer Blutbehandlungstherapie. In diesem Zustand ist es wünschenswert, das in dem extrakorporalen Kreislauf 4 noch vorhandene Blut dem Patienten zurückzugeben/ zu reinfundieren.

In dem in Fig. 1 gezeigten Ausführungsbeispiel kann optional nach Abschluss der Blutbehandlungstherapie und vor Beginn der Reinfusion ein Sicherheits-Luftentfernungsschritt durchgeführt werden. In dem in Fig. 2 gezeigten Ausführungsbeispiel ist dieser Sicherheits-Luftentfernungsschritt zwingend erforderlich. Grund dafür ist, dass in dem in Fig. 2 gezeigten Ausführungsbeispiel kein Sicherheitsluftdetektor in dem arteriellen Abschnitt 12 vorhanden ist.

Die Steuereinheit 46 führt den Sicherheits-Luftentfernungsschritt derart durch, dass sie zunächst den arteriellen Abschnitt 12 für etwa zwei Sekunden über die arterielle Schlauchklemme 22 abklemmt, während die arterielle Blutpumpe 26 in Therapierichtung weiterläuft. Dabei entsteht ein Unterdruck in dem arteriellen Abschnitt 12, welcher eine möglicherweise vorhandene Luft oder Luftblase 50 mitreißt und in den venösen Abschnitt 14 befördert, wo die Luft oder Luftblase 50 in der venösen Expansionskammer 16 eliminiert wird.

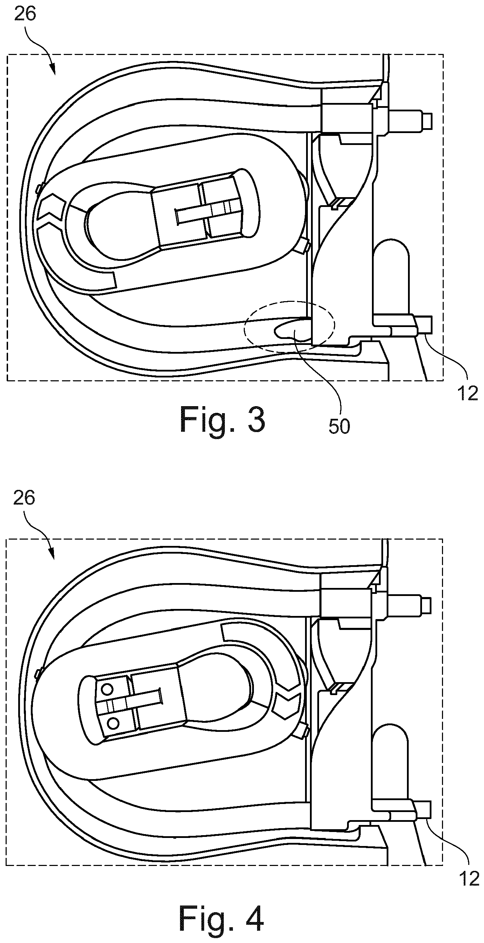

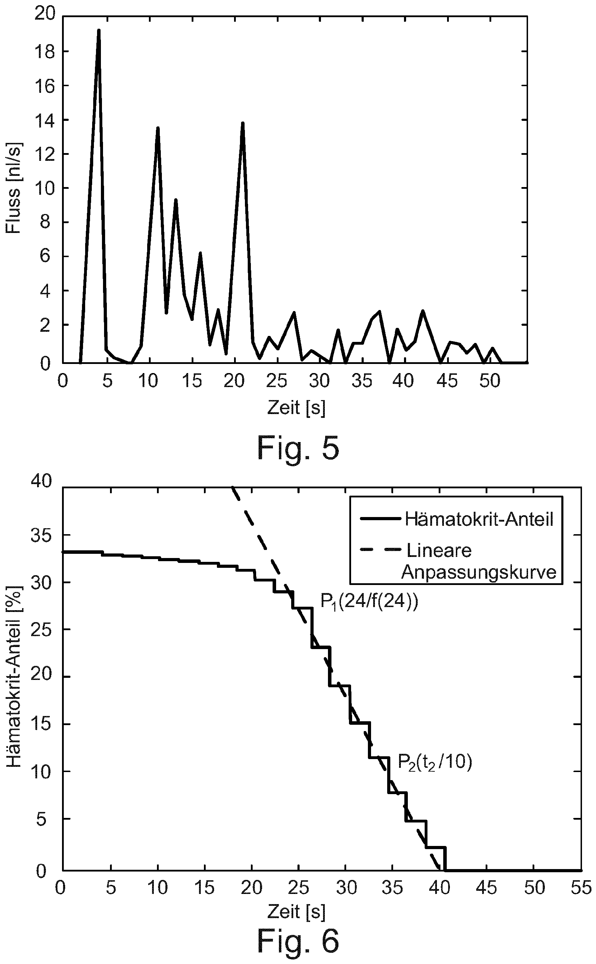

Wie aus Fig. 3 hervorgeht, befindet sich nach Abschluss einer Blutbehandlungstherapie oft eine Luftblase 50 an einem Eingang der arteriellen Blutpumpe 26. Eine solche Luftblase 50 kann durch den offenbarungsgemäßen Sicherheits-Luftentfernungsschritt in geeigneter Weise entfernt werden. Insbesondere zeigt Fig. 4 die arterielle Blutpumpe 26, nachdem der angesprochene Sicherheits-Luftentfernungsschritt durchgeführt wurde. Eine Luftblase 50 ist in Fig. 4 nicht mehr vorhanden und wurde durch den Sicherheits-Luftentfernungsschritt in geeigneter Weise eliminiert. Dies wird weiterhin auch durch das in Fig. 5 gezeigte Diagramm verdeutlicht. In Fig. 5 ist das Volumen/ der Volumenstrom/ Fluss von Luftbläschen/ Mikrobläschen über die Zeit bei der arteriellen Reinfusion dargestellt, nachdem der Sicherheits-Luftentfernungsschritt durchgeführt wurde. Es ist gezeigt, dass das Volumen/ der Fluss der Luftbläschen maximal 19 nl/s beträgt. Diese Flussrate von Luftbläschen liegt weit unter einer reglementierten maximalen Flussrate (500 nl/(s*kg)), so dass der offenbarungsgemäße Sicherheits-Luftentfernungsschritt ein zuverlässiges Entfernen der Luft ermöglicht.

Die Steuereinheit 46 ist vorliegend grundsätzlich eingerichtet, eine Reinfusion von Blut nach der Blutbehandlungstherapie bzw. gegebenenfalls nach dem Sicherheits-Luftentfernungsschritt automatisch derart zu steuern, dass eine Dialysierflüssigkeit von dem Dialysierflüssigkeitskreislauf 8 über die Membran 10 des Dialysators 6 an den extrakorporalen Kreislauf 4 geliefert wird, welche bei der Reinfusion das in dem extrakorporalen Kreislauf 4 vorhandene Blut zu dem Patienten 15 hin verdrängt, um das Blut dem Patienten 15 sowohl über den venösen Abschnitt 14 als auch über den arteriellen Abschnitt 12 zurückzugeben.

Die Steuereinheit 46 steuert dazu gemäß der vorliegenden Offenbarung in dem Dialysierflüssigkeitskreislauf 8 vorhandene Komponenten an, um einen Überdruck in dem Dialysierflüssigkeitskreislauf 8 zu erzeugen, welcher bewirkt, dass Dialysierflüssigkeit/ Reinfusionsflüssigkeit über die Membran 10 des Dialysators 6 tritt. Der Überdruck wird dabei bevorzugt erzeugt, indem die Steuereinheit 46 die Flusspumpe-Eingang 38 ansteuert, damit diese Dialysierflüssigkeit in den Dialysator 6 pumpt, während das Dialysatoreinlassventil 34 geöffnet ist, das Dialysatorauslassventil 36 geschlossen ist und die Flusspumpe-Ausgang 40 gestoppt ist. Es wird somit durch die Dialysierflüssigkeit ein geeigneter Druck (Transmembrandruck) auf die Membran 10 des Dialysators 6 ausgeübt. Dieser Druck bewirkt, dass Dialysierflüssigkeit mit einem definierten Fluss (Flussrate/ Volumenstrom), beispielsweise 100 ml/min (abhängig von dem verwendeten Dialysator) über die Membran 10 des Dialysators 6 von dem Dialysierflüssigkeitskreislauf 8 zu dem extrakorporalen Kreislauf 4 übertritt.

Grundsätzlich kann die Steuereinheit 46 die Steuerung in Abhängigkeit des verwendeten Dialysators 6 durchführen. Dazu klassifiziert die Steuereinheit 46 den verwendeten Dialysator 6 beispielsweise als High-Flux-Dialysator oder als Low-Flux-Dialysator und führt auf der Grundlage dieser Klassifizierung, insbesondere durch entsprechendes Ansteuern der Flusspumpe-Eingang 38, eine High-Flux-Steuerung oder eine Low-Flux-Steuerung durch.

Gemäß der vorliegenden Offenbarung kann die Steuereinheit 46 die Reinfusion über den arteriellen Abschnitt 12 und über den venösen Abschnitt 14 parallel oder seriell durchführen.

Bei der parallelen Reinfusion wird die arterielle Blutpumpe 26 von der Steuereinheit 46 entgegen der Therapierichtung betrieben, damit diese Blut/ Flüssigkeit von dem Dialysator 6 hin zu dem Ende des arteriellen Abschnitts 12 fördert. Dazu kann die Blutpumpe 26 von der Steuereinheit 46 auf eine konstante Förderrate eingestellt werden, welche geringer ist als der Fluss über die Membran 10 des Dialysators 6. Beispielsweise kann die Förderrate der Blutpumpe 26 auf 50 ml/min eingestellt werden. Wenn der definierte Fluss über die Membran 10 des Dialysators 6 100 ml/min ist, entspricht der Fluss in dem venösen Abschnitt 14 in Therapierichtung hin zu dem Ende des venösen Abschnitts 14 dem arteriellen Fluss. Wenn nun sowohl die arterielle Schlauchklemme 22 als auch die venöse Schlauchklemme 20 geöffnet sind, kann das Blut sowohl in dem arteriellen Abschnitt 12 als auch in dem venösen Abschnitt 14 des extrakorporalen Kreislaufs 4 durch die übertretende Dialysierflüssigkeit zum Patienten 15 hin verdrängt werden. Die parallele Reinfusion wird beendet, wenn sowohl die arterielle Reinfusion als auch die venöse Reinfusion von der Steuereinheit 46 abgebrochen wurden (durch Schließen der arteriellen Schlauchklemme 22 und der venösen Schlauchklemme 20).

Wenn bei der parallelen Reinfusion eine Blutreinfusion über den venösen Abschnitt 14 früher abgebrochen wird als eine Blutreinfusion über den arteriellen Abschnitt 12, schließt die Steuereinheit 46 die venöse Schlauchklemme 20 und erhöht die Förderrate der arteriellen Blutpumpe 26. Bei einem früheren Abbruch der Reinfusion über den arteriellen Abschnitt 12 stoppt die Steuereinheit 46 die arterielle Blutpumpe 26 und schließt die arterielle Schlauchklemme 22, so dass die übertretende Dialysierflüssigkeit nun nur noch das restliche Blut in dem venösen Abschnitt 14 verdrängt.

Bei der seriellen Reinfusion kann beispielsweise zunächst das Blut aus dem venösen Abschnitt 14 dem Patienten 15 zurückgegeben werden, und anschließend erst das Blut aus dem arteriellen Abschnitt 12 reinfundiert werden. In diesem Fall wird die arterielle Blutpumpe 26 angehalten, die venöse Schlauchklemme 20 geöffnet, und das Blut in dem venösen Abschnitt 14 durch die übertretende Dialysierflüssigkeit zum Patienten 15 hin verdrängt. Nach einem Abbruch der venösen Reinfusion (durch Schließen der venösen Schlauchklemme 20) wird die arterielle Schlauchklemme 22 geöffnet (oder bleibt geöffnet) und die arterielle Blutpumpe 26 beginnt, sich entgegen der Therapierichtung zu drehen, so dass nun auch das Blut aus dem arteriellen Abschnitt 12 dem Patienten 15 zurückgegeben wird. Die Förderrate der arteriellen Blutpumpe 26 kann dabei auf den Fluss der durch die Membran 10 übertretenden Dialysierflüssigkeit eingestellt werden. Durch einen Abbruch der arteriellen Reinfusion wird der serielle Reinfusionsvorgang beendet.

Alternativ kann bei der seriellen Reinfusion auch zunächst Blut aus dem arteriellen Abschnitt 12 dem Patienten 15 zurückgegeben werden, und anschließend erst das Blut aus dem venösen Abschnitt 14 reinfundiert werden. In diesem Fall wird zunächst die venöse Schlauchklemme 20 geschlossen bzw. bleibt geschlossen, die arterielle Schlauchklemme 22 wird geöffnet bzw. bleibt geöffnet, und die arterielle Blutpumpe 26 beginnt, sich entgegen der Therapierichtung zu drehen, so dass das Blut aus dem arteriellen Abschnitt 12 dem Patienten 15 zurückgegeben wird. Die Förderrate der arteriellen Blutpumpe 26 wird dabei auf den Fluss der durch die Membran 10 übertretenden Dialysierflüssigkeit eingestellt. Nach einem Abbruch der arteriellen Reinfusion (beispielsweise durch ein Anhalten der arteriellen Blutpumpe 26/ ein Schließen der arteriellen Schlauchklemme 22), wird die venöse Schlauchklemme 20 geöffnet, und das Blut wird in dem venösen Abschnitt 14 durch die übertretende Dialysierflüssigkeit zum Patienten 15 hin verdrängt. Durch einen Abbruch der venösen Reinfusion wird der serielle Reinfusionsvorgang insgesamt beendet.

Im Nachfolgenden wird genauer auf das erste bevorzugte Ausführungsbeispiel der vorliegenden Offenbarung gemäß Fig. 1 eingegangen. Gemäß diesem Ausführungsbeispiel ist sowohl in dem arteriellen Abschnitt 12 als auch in dem venösen Abschnitt 14 jeweils ein Sicherheitsluftdetektor mit integriertem Rotdetektor vorgesehen, nämlich der arterielle Sicherheitsluftdetektor mit integriertem Rotdetektor 24 und der venöse Sicherheitsluftdetektor mit integriertem Rotdetektor 18. Diese beiden Sicherheitsluftdetektoren mit integrierten Rotdetektoren 18, 24 erkennen Luft oder Luftblasen zuverlässig. Wird Luft bzw. werden Luftblasen erkannt, bricht die Steuereinheit gemäß diesem Ausführungsbeispiel die Reinfusion durch Stoppen der arteriellen Blutpumpe 26 oder durch Schließen der arteriellen Schlauchklemme 22 oder der venösen Schlauchklemme 20 ab.

Die Sicherheitsluftdetektoren mit integrierten Rotdetektoren 18, 24 sind eingerichtet, einen Hämatokrit-Anteil auch bei relativ kleinen prozentualen Werten noch recht genau zu bestimmen. Gemäß dem ersten bevorzugten Ausführungsbeispiel wird die arterielle Reinfusion abgebrochen, wenn der arterielle Sicherheitsluftdetektor mit integriertem Rotdetektor 24 erfasst, dass der Hämatokrit-Anteil in einem Endbereich des arteriellen Abschnitts 12 unter dem vorbestimmten Grenzwert liegt, welcher bevorzugt 3% ist. Die venöse Reinfusion wird abgebrochen, wenn der venöse Sicherheitsluftdetektor mit integriertem Rotdetektor 18 erfasst, dass der Hämatokrit-Anteil in einem Endbereich des venösen Abschnitts 12 unter dem vorbestimmten Grenzwert liegt.

Im Nachfolgenden wird noch genauer auf das zweite bevorzugte Ausführungsbeispiel der vorliegenden Offenbarung gemäß Fig. 2 eingegangen. Gemäß diesem Ausführungsbeispiel ist in dem venösen Abschnitt 14 der venöse Sicherheitsluftdetektor mit integriertem Rotdetektor 18 und in dem arteriellen Abschnitt 12 der Hämatokritsensor/ HTC-Sensor 48 vorgesehen.

Da der Hämatokritsensor 48 einen Hämatokrit-Wert/Anteil lediglich in einem Bereich zwischen 20% und 55% zuverlässig messen kann, der vorbestimmte Grenzwert offenbarungsgemäß bei kleiner 10%, insbesondere bei 3%, liegt, werden die Messergebnisse des Hämatokritsensors 48 offenbarungsgemäß von der Steuereinheit 46 intelligent ausgewertet, um vorhersagen/ berechnen zu können, wann der vorbestimmte Grenzwert unterschritten wird.

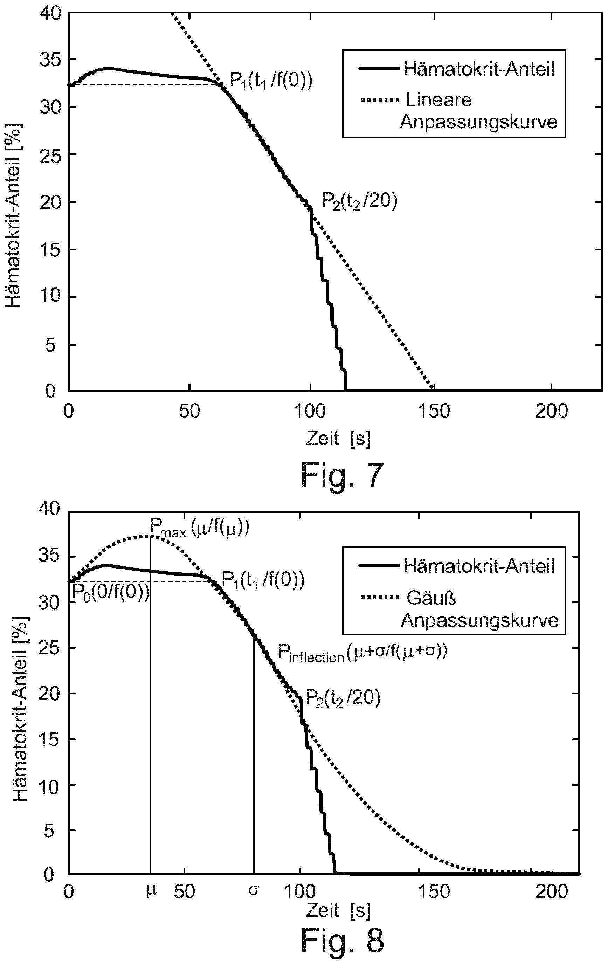

Eine derartige intelligente Auswertung wird für die serielle Reinfusion (Blutrückgabe über sowohl den arteriellen Abschnitt 12 als auch den venösen Abschnitt 14 mit einer Flussrate von 100 ml/min) unter Bezugnahme auf das in Fig. 6 dargestellte Diagramm veranschaulicht. Für die parallele Reinfusion (Blutrückgabe über sowohl den arteriellen Abschnitt 12 als auch den venösen Abschnitt 14 mit einer Flussrate von 50 ml/min) wird eine derartige intelligente Auswertung unter Bezugnahme auf die Diagramme in Fig. 7 und Fig. 8 veranschaulicht.

Um den Hämatokritsensor 48 intelligent auswerten zu können, muss ein Messverlauf des Hämatokritsensors 48 mit einem tatsächlichen Verlauf des Hämatokrit-Anteils verglichen werden. Zu Versuchszwecken wurde dazu ein SADRDV-Sensor als Referenzsensor in den arteriellen Abschnitt 12 integriert. Für die nachfolgend beschriebenen Versuche wurde der High-Flux-Dialysator "Xevonta Hi 23" verwendet.

In Fig. 6 ist für die serielle Reinfusion lediglich der von dem Hämatokritsensor 48 gemessene Verlauf des Hämatokrit-Anteils für die arterielle Blutreinfusion gezeigt. Bei dem Versuch fasste das Blutschlauchsystem zwischen dem Hämatokritsensor 48 und dem Dialysator 6 etwa 40 ml. Bei einem Fluss von 100 ml/min ergibt sich, dass das Blut etwa 24 Sekunden lang relativ unverdünnt den Hämatokritsensor 48 passiert (nur leichte Abnahme des Hämatokrit-Anteils in Fig. 6). Nach 24 Sekunden nimmt der Hämatokrit-Anteil stark ab, bis er innerhalb von 17 Sekunden einen Wert von 0% erreicht (gemäß Hämatokritsensor 48). Wenn der Hämatokritsensor 48 die starke Abnahme des Hämatokrit-Anteils erfasst, kann ein Punkt P1 definiert werden. Ein zweiter Punkt P2 kann definiert werden, wenn der durch den Hämatokritsensor 48 gemessene Hämatokrit-Anteil sich bei einem prozentualen Wert befindet, bei welchem der Hämatokritsensor 48 noch genau misst, also beispielsweise bei 20%. In Fig. 6 wurde P2 bei einem Hämatokrit-Anteil von 10% definiert. Durch die Punkte P1 und P2 wird eine lineare Funktion f(t) = -1,8 * t + 72,6 (für t > 24 s) definiert. Für f(t) = 0 ergibt sich somit eine Zeit von 40 Sekunden. Aufgrund der Referenzmessung mit dem SADRDV-Sensor ist es jedoch bekannt, dass der Hämatokrit-Anteil nach 55 Sekunden erst den vorbestimmten Grenzwert von 3% unterschreitet, woraus sich ein Offset (für den verwendeten Dialysator, den eingestellten Fluss, und das verwendete Schlauchsystem) von 15 Sekunden ergibt. Vor diesem Hintergrund kann die Steuereinheit 46 zur intelligenten Auswertung der Messergebnisse des HämatokritSensors die Punkte P1 und P2 definieren, eine lineare Funktion f(t), welche durch die Punkte P1 und P2 verläuft, definieren, und wenn gemäß der linearen Funktion f(t) = 0 wird, gegebenenfalls noch einen Offset dazurechnen, um zu bestimmen, wann der vorbestimmte Grenzwert unterschritten wird. Diese Annäherung funktioniert jedoch nur, wenn der Offset gering ist (insbesondere kleiner als 20 Sekunden).

Gemäß Fig. 7 wurde versucht, auch bei der parallelen Reinfusion durch Anlegen einer linearen Funktion an den Messverlauf des Hämatokritsensors 48, zu bestimmen, wann der vorbestimmte Grenzwert unterschritten wird. Wie aus Fig. 7 hervorgeht, unterscheidet sich der Verlauf des von dem Hämatokritsensor 48 gemessenen Verlaufs des Hämatokrit-Anteils bei der parallelen Reinfusion von dem der seriellen Reinfusion. Insbesondere nimmt der Hämatokrit-Anteil in Fig. 7 während der ersten 63 Sekunden nicht signifikant ab, da das Blut im arteriellen Abschnitt 12 des extrakorporalen Kreislaufs 4 den Hämatokritsensor 48 relativ unverdünnt passiert. Da ein Fluss von lediglich 50 ml/min anliegt, dauert dieser Vorgang etwa doppelt so lange wie bei der seriellen Reinfusion. Danach sinkt der Hämatokrit-Anteil mit einem konstanten Gradienten auf 20% innerhalb von 34 Sekunden ab. Es folgt ein rascher Rückgang auf einen Hämatokrit-Anteil von 0% innerhalb von 18 Sekunden. Insgesamt ist somit eine unterschiedliche Abnahme des Hämatokrit-Anteils im Wertebereich von 33% bis 17% und im Wertebereich von 17% bis 0% zu beobachten. Vor diesem Hintergrund kann angenommen werden, dass der zuverlässige Messbereich des Hämatokritsensors 48 nur bis etwa 20% reicht. In diesem Bereich wurde in Fig. 7 eine lineare Funktion angelegt, mit folgender Funktionsgleichung: f(t) = -0,36*t + 54,23 [t>48 Sekunden]. Für diese Funktion gilt, dass f(t) = 0 bei 156 Sekunden vorliegt. Die tatsächliche mit dem Referenzsensor gemessene Zeit, bis der vorbestimmte Grenzwert von 3% unterschritten wird, beträgt vorliegend jedoch 221 Sekunden. Vor diesem Hintergrund ist bei der parallelen Reinfusion der Offset zu groß, um ein Unterschreiten des vorbestimmten Grenzwerts mit einer linearen Funktion vorherzusagen.

Wie aus Fig. 8 hervorgeht, hat sich offenbarungsgemäß herausgestellt, dass eine Gauß-Funktion erster Ordnung für die Vorhersage/ Berechnung, wann der Hämatokrit-Anteil den vorbestimmten Grenzwert unterschreitet, bei der parallelen Reinfusion verwendet werden kann. Insbesondere kann die Gauß-Funktion hergeleitet werden, wenn der Hämatokrit-Anteil von dem Hämatokritsensor 48 bis 20% aufgezeichnet wird. Insbesondere werden drei Punkte P0, P1 und P2 des Hämatokrit-Verlaufes für die Herleitung der Gauß-Funktion verwendet, wobei P0 der Startpunkt ist, P1 der Punkt ist, ab welchem der Hämatokrit-Anteil mit einem konstanten Gradienten absinkt, und P2 der Punkt ist, bis zu welchem der Hämatokrit-Sensor zuverlässig misst (etwa bei 20%). Wenn nun das Maximum Pmax der Gauß-Funktion derart positioniert wird, dass es sich auf der Zeitachse genau in der Mitte zwischen P0 und P1 befindet, und der Wendepunkt Pinflection der Gauß-Funktion derart definiert wird, dass er sich genau in der Mitte der Strecke zwischen P1 und P2 befindet, kann wie in Fig. 8 gezeigt die Gauß-Funktion wie folgt definiert werden:

$$f(t) = 37{,}3 * \exp\left[-\left(\frac{t - 31{,}1}{57{,}1}\right)^2\right]$$

Wie aus Fig. 8 hervorgeht, gilt für diese Funktion, dass f(t) = 0 bei etwa 216 Sekunden vorliegt. Somit kann mit dieser Funktion in geeigneter Weise vorhergesagt werden, insbesondere wenn ein geringer Offset hinzugerechnet wird, wann der vorbestimmte Grenzwert von 3% unterschritten wird.

Bezugszeichenliste

| | |
|---|---|
| 2 | extrakorporale Blutbehandlungsvorrichtung |
| 4 | extrakorporaler Kreislauf |
| 6 | Dialysator |
| 8 | Dialysierflüssigkeitskreislauf |
| 10 | Membran |
| 12 | arterieller Abschnitt |
| 14 | venöser Abschnitt |
| 15 | Patient |
| 16 | venöse Expansionskammer/ Luftfalle |
| 18 | venöser Sicherheitsluftdetektor mit integriertem Rotdetektor (SADRDV-Sensor) |
| 20 | venöse Schlauchklemme |
| 22 | arterielle Schlauchklemme |
| 24 | arterieller Sicherheitsluftdetektor mit integriertem Rotdetektor (SADRDV-Sensor) |
| 26 | (arterielle) Blutpumpe |
| 28 | arterieller Drucksensor |
| 30 | Dialysatoreingangsdrucksensor |
| 32 | venöser Drucksensor |
| 34 | Dialysatoreinlassventil |

36 Dialysatorauslassventil
38 Flusspumpe-Eingang
40 Flusspumpe-Ausgang
42 Dialysierflüssigkeitszufluss
44 Dialysierflüssigkeitsabfluss
46 Steuereinheit
48 Hämatokritsensor (HTC-Sensor)
50 Luftblase

**Patentansprüche**

1. Extrakorporale Blutbehandlungsvorrichtung (2) mit:

einem extrakorporalen Kreislauf (4) umfassend einen arteriellen Abschnitt (12) und einen venösen Abschnitt (14), einem Dialysator (6) und einem Dialysierflüssigkeitskreislauf (8), wobei der extrakorporale Kreislauf (4) und der Dialysierflüssigkeitskreislauf (8) über eine in dem Dialysator (6) vorgesehene Membran (10) voneinander getrennt sind;
in dem venösen Abschnitt (14) ein erster Sensor (18) vorgesehen ist, welcher eingerichtet ist, einen Hämatokrit-Anteil in einer den venösen Abschnitt (14) durchströmenden Flüssigkeit zu erfassen;
in dem arteriellen Abschnitt (12) ein zweiter Sensor (24, 48) vorgesehen ist, welcher eingerichtet ist, einen Hämatokrit-Anteil in einer den arteriellen Abschnitt (12) durchströmenden Flüssigkeit zu erfassen; und
die extrakorporale Blutbehandlungsvorrichtung (2) ferner eine Steuereinheit (46) enthält, welche eingerichtet ist, eine Reinfusion von Blut derart zu steuern, dass eine Dialysierflüssigkeit von dem Dialysierflüssigkeitskreislauf (8) über die Membran (10) des Dialysators (6) an den extrakorporalen Kreislauf (4) geliefert wird, welche bei der Reinfusion das in dem extrakorporalen Kreislauf (4) vorhandene Blut zu einem Patienten (15) hin verdrängt, um das Blut dem Patienten (15) sowohl über den venösen Abschnitt (14) als auch über den arteriellen Abschnitt (12) zurückzugeben,
**dadurch gekennzeichnet, dass**
die Steuereinheit (46) eingerichtet ist, die Reinfusion über den venösen Abschnitt (14) abzubrechen, wenn von dem ersten Sensor (18) erfasst wird oder von der Steuereinheit (46) auf der Grundlage von von dem ersten Sensor (18) erfassten Informationen berechnet oder vorhergesagt wird, dass der Hämatokrit-Anteil einen vorbestimmten Grenzwert unterschreitet, und die Reinfusion über den arteriellen Abschnitt (12) abzubrechen, wenn von dem zweiten Sensor (24, 48) erfasst wird oder von der Steuereinheit (46) auf der Grundlage von von dem zweiten Sensor (24, 48) erfassten Informationen berechnet oder vorhergesagt wird, dass der Hämatokrit-Anteil den vorbestimmten Grenzwert unterschreitet.

2. Extrakorporale Blutbehandlungsvorrichtung (2) nach Anspruch 1, **dadurch gekennzeichnet, dass** der vorbestimmte Grenzwert für den Hämatokrit-Anteil bei kleiner oder gleich 10%, bevorzugt zwischen 1 % und 5%, besonders bevorzugt bei 3% liegt.

3. Extrakorporale Blutbehandlungsvorrichtung (2) nach Anspruch 1 oder 2, **dadurch gekennzeichnet, dass** in dem arteriellen Abschnitt (12) eine arterielle Blutpumpe (26) vorgesehen ist, welche für einen Drehrichtungswechsel eingerichtet ist, und die Steuereinheit (46) bei der Reinfusion über den arteriellen Abschnitt (12) die arterielle Blutpumpe (26) ansteuert, damit sich diese entgegen einer Therapierichtung dreht.

4. Extrakorporale Blutbehandlungsvorrichtung (2) nach einem der vorhergehenden Ansprüche 1 bis 3, **dadurch gekennzeichnet, dass** die Steuereinheit (46) eingerichtet ist, die Reinfusion über den venösen Abschnitt (14) und die Reinfusion über den arteriellen Abschnitt (12) parallel durchzuführen.

5. Extrakorporale Blutbehandlungsvorrichtung (2) nach einem der Ansprüche 1 bis 3, **dadurch gekennzeichnet, dass** die Steuereinheit (46) eingerichtet ist, die Reinfusion über den venösen Abschnitt (14) und die Reinfusion über den arteriellen Abschnitt (12) seriell durchzuführen.

6. Extrakorporale Blutbehandlungsvorrichtung (2) nach einem der vorhergehenden Ansprüche 1 bis 5, **dadurch gekennzeichnet, dass** die Steuereinheit (46) eingerichtet ist, die Reinfusion in Abhängigkeit von dem verwendeten Dialysator (6) zu steuern.

7. Extrakorporale Blutbehandlungsvorrichtung (2) nach einem der vorhergehenden Ansprüche 1 bis 6, **dadurch gekennzeichnet, dass** die Steuereinheit (46) eingerichtet ist, nach der Blutbehandlungstherapie und vor der Reinfusion einen Sicherheits-Luftentfernungsschritt durchzuführen, mittels welchem Luft bzw. eine Luftblase aus dem arteriellen Abschnitt (12) entfernt wird.

8. Extrakorporale Blutbehandlungsvorrichtung (2) nach Anspruch 7, **dadurch gekennzeichnet, dass** in dem arteriellen Abschnitt (12) eine arterielle Schlauchklemme (22) und eine/die arterielle Blutpumpe (26) vorgesehen sind, und in dem venösen Abschnitt (14) eine venöse Expansionskammer (16) vorgesehen ist, und die Steuereinheit (46) den Sicherheits-Luftentfernungsschritt derart durchführt, dass zunächst der arterielle Abschnitt (12) für eine vorbestimmte Zeitdauer über die arterielle Schlauchklemme (22) abgeklemmt wird, während die arterielle Blutpumpe (26) in Therapierichtung weiterläuft, wobei der dadurch entstehende Unterdruck die in dem arteriellen Abschnitt (12) vorhandene Luft bzw. Luftblase (50) mitreißt und in den venösen Abschnitt (14) befördert, wo sie in der venösen Expansionskammer (16) eliminiert wird.

9. Extrakorporale Blutbehandlungsvorrichtung (2) nach einem der vorhergehenden Ansprüche, **dadurch gekennzeichnet, dass** der erste Sensor (18) ein venöser Sicherheitsluftdetektor mit integriertem Rotdetektor (18) ist, welcher eingerichtet ist, Luft oder Luftblasen in der den venösen Abschnitt (14) durchströmenden Flüssigkeit zu erkennen und den Hämatokrit-Anteil der Flüssigkeit als Abbruchkriterium der Reinfusion über den venösen Abschnitt (14) zu erfassen, und dass der zweite Sensor (24, 48) ein arterieller Sicherheitsluftdetektor mit integriertem Rotdetektor (24) ist, welcher eingerichtet ist, Luft oder Luftblasen in der den arteriellen Abschnitt (12) durchströmenden Flüssigkeit zu erkennen und den Hämatokrit-Anteil der Flüssigkeit als Abbruchkriterium der Reinfusion über den arteriellen Abschnitt (12) zu erfassen.

10. Extrakorporale Blutbehandlungsvorrichtung (2) nach Anspruch 7 oder 8, **dadurch gekennzeichnet, dass** der erste Sensor (18) ein venöser Sicherheitsluftdetektor mit integriertem Rotdetektor (18) ist, welcher eingerichtet ist, Luft oder Luftblasen in der den venösen Abschnitt (14) durchströmenden Flüssigkeit zu erkennen und den Hämatokrit-Anteil der Flüssigkeit als Abbruchkriterium der Reinfusion über den venösen Abschnitt (14) zu erfassen, und dass der zweite Sensor ein Hämatokrit-Sensor (48) ist, welcher eingerichtet ist, Informationen über den Hämatokrit-Anteil in der Flüssigkeit während der Reinfusion zu erfassen und diese Informationen an die Steuereinheit (46) weiterzugeben.

11. Extrakorporale Blutbehandlungsvorrichtung (2) nach Anspruch 10, **dadurch gekennzeichnet, dass** die Steuereinheit (46) eingerichtet ist, auf der Grundlage der Informationen über den Hämatokrit-Anteil in der Flüssigkeit eine Berechnung oder eine Vorhersage dahingehend vorzunehmen, wann der Hämatokrit-Anteil der Flüssigkeit den vorbestimmten Grenzwert unterschreitet.

12. Extrakorporale Blutbehandlungsvorrichtung (2) nach Anspruch 11, **dadurch gekennzeichnet, dass** die Steuereinheit (46) eingerichtet ist, die Berechnung oder die Vorhersage mittels einer linearen Funktion vorzunehmen, wenn sie die Reinfusion über den venösen Abschnitt (14) und die Reinfusion über den arteriellen Abschnitt (12) seriell durchführt oder wenn ein Reinfusionsfluss in dem arteriellen Abschnitt (12) oder dem venösen Abschnitt (14) groß ist bzw. größer einem vorbestimmten Schwellwert ist.

13. Extrakorporale Blutbehandlungsvorrichtung (2) nach Anspruch 11 oder 12, **dadurch gekennzeichnet, dass** die Steuereinheit (46) eingerichtet ist, die Berechnung oder die Vorhersage mittels einer Gauß-Funktion vorzunehmen, wenn sie die Reinfusion über den venösen Abschnitt (14) und die Reinfusion über den arteriellen Abschnitt (12) parallel durchführt oder wenn ein Reinfusionsfluss in dem arteriellen Abschnitt (12) oder dem venösen Abschnitt (14) klein ist bzw. kleiner einem vorbestimmten Schwellwert ist.

**Claims**

1. Extracorporeal blood treatment device (2) comprising:

    an extracorporeal circuit (4) comprising an arterial section (12) and a venous section (14), a dialyzer (6) and a dialyzing liquid circuit (8), wherein
    the extracorporeal circuit (4) and the dialyzing liquid circuit (8) are separated from each other by a membrane (10) provided in the dialyzer (6);
    in the venous section (14) a first sensor (18) is provided, which is configured to acquire a hematocrit percentage in a liquid flowing through the venous section (14);
    in the arterial section (12) a second sensor (24, 48) is provided, which is configured to acquire a hematocrit percentage in a liquid flowing through the arterial section (12); and
    the extracorporeal blood treatment device (2) further comprises a control unit (46) which is configured to control a reinfusion of blood such that a dialyzing liquid is supplied from the dialyzing liquid circuit (8) via the membrane (10) of the dialyzer (6) to the extracorporeal circuit (4), which during the reinfusion displaces the blood present in the extracorporeal circuit (4) towards a patient (15) to return the blood to the patient (15) via both the venous section (14) and the arterial section (12),
    **characterized in that**
    the control unit (46) is configured to discontinue the reinfusion via the venous section (14) if it is acquired by the first sensor (18) or calculated or predicted by the control unit (46) on the basis of information acquired by the first sensor (18) that the hematocrit percentage falls below a predetermined limit value, and to discontinue the reinfusion via the arterial section (12) if it is acquired by the second sensor (24, 48) or calculated or predicted by the control unit (46) on the basis of information acquired by the second sensor (24, 48) that the hematocrit percentage falls below the predetermined limit value.

2. Extracorporeal blood treatment device (2) according to claim 1, **characterized in that** the predetermined limit value for the hematocrit percentage is less than or equal to 10%, preferably between 1% and 5%, more preferably at 3%.

3. Extracorporeal blood treatment device (2) according to claim 1 or 2, **characterized in that** an arterial blood pump (26) is provided in the arterial section (12), which arterial blood pump (26) is configured for a change of direction of rotation, and the control unit (46) controls the arterial blood pump (26) during the reinfusion via the arterial section (12) so that the arterial blood pump (26) rotates against a therapy direction.

4. Extracorporeal blood treatment device (2) according to any of the preceding claims 1 to 3, **characterized in that** the control unit (46) is configured to in parallel perform the reinfusion via the venous section (14) and the reinfusion via the arterial section (12).

5. Extracorporeal blood treatment device (2) according to any of claims 1 to 3, **characterized in that** the control unit (46) is configured to serially perform the reinfusion via the venous section (14) and the reinfusion via the arterial section (12).

6. Extracorporeal blood treatment device (2) according to any of the preceding claims 1 to 5, **characterized in that** the control unit (46) is configured to control the reinfusion as a function of the dialyzer (6) used.

7. Extracorporeal blood treatment device (2) according to any of the preceding claims 1 to 6, **characterized in that** the control unit (46) is configured to perform, after the blood treatment therapy and before the reinfusion, a safety air removal step, by means of which air or, respectively, an air bubble is removed from the arterial section (12).

8. Extracorporeal blood treatment device (2) according to claim 7, **characterized in that** an arterial tube clamp (22) and an/the arterial blood pump (26) are provided in the arterial section (12), and a venous expansion chamber (16) is provided in the venous section (14), and the control unit (46) performs the safety air removal step such that initially the arterial section (12) is clamped off for a predetermined period of time via the arterial tube clamp (22), while the arterial blood pump (26) continues to run in therapy direction, wherein the thereby resulting negative pressure carries away the air or, respectively, air bubble (50) present in the arterial section (12) and conveys it into the venous section (14), where it is eliminated in the venous expansion chamber (16).

9. Extracorporeal blood treatment device (2) according to any of the preceding claims, **characterized in that** the first sensor (18) is a venous safety air detector with integrated red detector (18), which is configured to detect air or air bubbles in the liquid flowing through the venous section (14) and to acquire the hematocrit percentage of the liquid as termination criterion of the reinfusion via the venous section (14), and **in that** the second sensor (24, 48) is an arterial safety air detector with integrated red detector (24), which is configured to detect air or air bubbles in the liquid flowing through the arterial section (12) and to acquire the hematocrit percentage of the liquid as termination criterion of the reinfusion via the arterial section (12).

10. Extracorporeal blood treatment device (2) according to claim 7 or 8, **characterized in that** the first sensor (18) is a venous safety air detector with integrated red detector (18), which is configured to detect air or air bubbles in the liquid flowing through the venous section (14) and to acquire the hematocrit percentage of the liquid as termination criterion of the reinfusion via the venous section (14), and **in that** the second sensor is a hematocrit sensor (48) which is configured to acquire information about the hematocrit percentage in the liquid during the reinfusion and to forward this information to the control unit (46).

11. Extracorporeal blood treatment device (2) according to claim 10, **characterized in that** the control unit (46) is configured to make, on the basis of the information about the hematocrit percentage in the liquid, a calculation or a prediction as to when the hematocrit percentage of the liquid falls below the predetermined limit value.

12. Extracorporeal blood treatment device (2) according to claim 11, **characterized in that** the control unit (46) is configured to make the calculation or the prediction by means of a linear function when performing the reinfusion via the venous section (14) and the reinfusion via the arterial section (12) serially or when a reinfusion flow in the arterial section (12) or the venous section (14) is large or, respectively, is greater than a predetermined threshold value.

13. Extracorporeal blood treatment device (2) according to claim 11 or 12, **characterized in that** the control unit (46) is configured to make the calculation or the prediction by means of a Gaussian function when performing the reinfusion via the venous section (14) and the reinfusion via the arterial section (12) in parallel or when a reinfusion flow in the arterial section (12) or the venous section (14) is small or, respectively, is smaller than a predetermined threshold value.

**Revendications**

1. Dispositif de traitement du sang extracorporel (2), comportant :

   un circuit extracorporel (4) comprenant une section artérielle (12) et une section veineuse (14), un dialyseur (6) et un circuit de liquide de dialyse (8),
   le circuit extracorporel (4) et le circuit de liquide de dialyse (8) étant séparés l'un de l'autre par l'intermédiaire d'une membrane (10) prévue dans le dialyseur (6) ;
   un premier capteur (18) étant prévu dans la section veineuse (14), lequel étant configuré pour détecter un taux d'hématocrite dans un liquide circulant dans la section veineuse (14) ;
   un second capteur (24, 48) étant prévu dans la section artérielle (12), lequel étant configuré pour détecter un taux d'hématocrite dans un liquide circulant dans la section artérielle (12) ; et
   le dispositif de traitement du sang extracorporel (2) présentant en outre une unité de commande (46) configurée pour commander une reperfusion de sang de sorte qu'un liquide de dialyse est fourni au circuit extracorporel (4) à partir du circuit de liquide de dialyse (8) à travers la membrane (10) du dialyseur (6), lequel liquide de dialyse est déplacé en direction d'un patient (15) lors de la reperfusion du sang présent dans le circuit extracorporel (4) pour restituer le sang au patient (15) aussi bien à travers la section veineuse (14) qu'à travers la section artérielle (12), **caractérisé en ce que**
   l'unité de commande (46) est configurée pour interrompre la reperfusion à travers la section veineuse (14) lorsque le fait que le taux d'hématocrite est inférieur à une valeur limite prédéterminée est détecté par le premier capteur (18) ou est calculé ou prédit par l'unité de commande (46) sur la base d'informations détectées par le premier capteur (18), et pour interrompre la reperfusion à travers la section artérielle (12) lorsque le fait que le taux d'hématocrite est inférieur à la valeur limite prédéterminée est détecté par le second capteur (24, 48) ou est calculé ou prédit par l'unité de commande (46) sur la base d'informations détectées par le second capteur (24, 48).

2. Dispositif de traitement du sang extracorporel (2) selon la revendication 1, **caractérisé en ce que** la valeur limite prédéterminée pour le taux d'hématocrite est inférieure ou égale à 10 %, de préférence se situe entre 1 % et 5 %, de manière particulièrement préférée est de 3 %.

3. Dispositif de traitement du sang extracorporel (2) selon la revendication 1 ou 2, **caractérisé en ce qu'**une pompe à sang artérielle (26) est prévue dans la section artérielle (12), laquelle pompe à sang est conçue pour une inversion du sens de rotation, et, lors de la reperfusion à travers la section artérielle (12), l'unité de commande (46) commande la pompe à sang artérielle (26) afin que celle-ci tourne dans le sens inverse d'un sens de la thérapie.

4. Dispositif de traitement du sang extracorporel (2) selon l'une des revendications précédentes 1 à 3, **caractérisé en ce que** l'unité de commande (46) est configurée pour réaliser la reperfusion à travers la section veineuse (14) et la reperfusion à travers la section artérielle (12) simultanément.

5. Dispositif de traitement du sang extracorporel (2) selon l'une des revendications 1 à 3, **caractérisé en ce que** l'unité de commande (46) est configurée pour réaliser la reperfusion à travers la section veineuse (14) et la reperfusion à travers la section artérielle (12) l'une après l'autre.

6. Dispositif de traitement du sang extracorporel (2) selon l'une des revendications précédentes 1 à 5, **caractérisé en ce que** l'unité de commande (46) est configurée pour commander la reperfusion en fonction du dialyseur (6) utilisé.

7. Dispositif extracorporel de traitement du sang (2) selon l'une des revendications précédentes 1 à 6, **caractérisé en ce que** l'unité de commande (46) est configurée pour réaliser, après la thérapie de traitement du sang et avant la reperfusion, une étape d'élimination d'air pour la sécurité au moyen de laquelle de l'air ou une bulle d'air est retiré de la section artérielle (12).

8. Dispositif de traitement du sang extracorporel (2) selon la revendication 7, **caractérisé en ce qu'**un collier de serrage artériel (22) et une/la pompe à sang artérielle (26) sont prévus dans la section artérielle (12), et une chambre d'expansion veineuse (16) est prévue dans la section veineuse (14), et l'unité de commande (46) réalise l'étape d'élimination d'air pour la sécurité de sorte que la section artérielle (12) en premier lieu est serrée par l'intermédiaire du collier de serrage artériel (22) pendant une durée prédéterminée tandis que la pompe à sang artérielle (26) poursuit son fonctionnement dans le sens de la thérapie, la dépression qui en résulte entraînant l'air ou la bulle d'air (50) présent dans la section artérielle (12) et le transportant vers la section veineuse (14) où il est éliminé dans la chambre d'expansion veineuse (16).

9. Dispositif de traitement du sang extracorporel (2) selon l'une des revendications précédentes, **caractérisé en ce que** le premier capteur (18) est un détecteur d'air pour la sécurité veineux comportant un détecteur rouge (18) intégré, lequel est configuré pour identifier de l'air ou des bulles d'air dans le liquide circulant dans la section veineuse (14) et pour détecter le taux d'hématocrite dans le liquide comme critère d'interruption de la reperfusion à travers la section veineuse (14), **et en ce que** le second capteur (24, 48) est un détecteur d'air pour la sécurité veineux comportant un détecteur rouge (24) intégré, lequel est configuré pour identifier de l'air ou des bulles d'air dans le liquide circulant dans la section artérielle (12) et pour détecter le taux d'hématocrite dans le liquide comme critère d'interruption de la reperfusion à travers la section artérielle (12).

10. Dispositif de traitement du sang extracorporel (2) selon la revendication 7 ou 8, **caractérisé en ce que** le premier capteur (18) est un détecteur d'air pour la sécurité veineux comportant un détecteur rouge (18) intégré, lequel est configuré pour identifier de l'air ou des bulles d'air dans le liquide circulant dans la section veineuse (14) et pour détecter le taux d'hématocrite dans le liquide comme critère d'interruption de la reperfusion à travers la section veineuse (14), **et en ce que** le second capteur est un capteur d'hématocrite (48), lequel est configuré pour détecter des informations concernant le taux d'hématocrite dans le liquide pendant la reperfusion et pour transmettre lesdites informations à l'unité de commande (46).

11. Dispositif de traitement du sang extracorporel (2) selon la revendication 10, **caractérisé en ce que** l'unité de commande (46) est configurée pour, sur la base des informations concernant le taux d'hématocrite dans le liquide, effectuer un calcul ou une prédiction concernant le moment où le taux d'hématocrite dans le liquide passera en dessous de la valeur limite prédéterminée.

12. Dispositif de traitement du sang extracorporel (2) selon la revendication 11, **caractérisé en ce que** l'unité de commande (46) est configurée pour effectuer le calcul ou la prédiction à l'aide d'une fonction linéaire lorsqu'elle réalise la reperfusion à travers la section veineuse (14) et la reperfusion à travers la section artérielle (12) l'une après l'autre ou lorsqu'un flux de reperfusion dans la section artérielle (12) ou dans la section veineuse (14) est élevé ou supérieur à une valeur seuil prédéterminée.

13. Dispositif de traitement du sang extracorporel (2) selon la revendication 11 ou 12, **caractérisé en ce que** l'unité de commande (46) est configurée pour effectuer le calcul ou la prédiction à l'aide d'une fonction gaussienne lorsqu'elle réalise la reperfusion à travers la section veineuse (14) et la reperfusion à travers la section artérielle (12) simultanément ou lorsqu'un flux de reperfusion dans la section artérielle (12) ou dans la section veineuse (14) est faible ou inférieur à une valeur seuil prédéterminée.

Fig. 1

Fig. 2

Fig. 3

Fig. 4

Fig. 5

Fig. 6

EP 4 065 191 B1

Fig. 7

Fig. 8

22

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1996253 B1
- EP 2950841 B1
- EP 2583702 B1
- EP 3476414 A1
- EP 1684825 B1
- DE 3535036 A1
- DE 102010025516 A1
- EP 2583701 A1
- US 2017361004 A1
- EP 3231466 B1